(19) Europäisches Patentamt
European Patent Office
Office européen des brevets

(11) **EP 4 223 223 A1**

(12) **EUROPEAN PATENT APPLICATION**

(43) Date of publication:
**09.08.2023 Bulletin 2023/32**

(21) Application number: **22382090.3**

(22) Date of filing: **02.02.2022**

(51) International Patent Classification (IPC):
***A61B 5/383*** (2021.01)    ***A61B 5/00*** (2006.01)

(52) Cooperative Patent Classification (CPC):
**A61B 5/383; A61B 5/4076; A61B 5/4848;
A61B 5/7275**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Fundació Institut d'Investigació en
Ciències de la
Salut Germans Trias i Pujol
08916 Badalona (ES)**

(72) Inventors:
• **MARTÍNEZ PIÑEIRO, Alicia**
  **08916 Badalona (ES)**
• **DÁVALOS ERRANDO, Antonio**
  **08916 Badalona (ES)**
• **COLL-CANTÍ, Jaume**
  **08916 Badalona (ES)**

(74) Representative: **Hoffmann Eitle
Hoffmann Eitle S.L.U.
Paseo de la Castellana 140, 3a planta
Edificio LIMA
28046 Madrid (ES)**

(54) **PREDICTION OF THE OUTCOME OF ENDOVASCULAR TREATMENT IN ACUTE ISCHEMIC STROKE PATIENTS**

(57)    Use of a somatosensory evoked potential, SEP, as a marker for predicting the outcome of an endovascular stroke treatment. The absolute amplitude of a local maximum or minimum of the SEP ipsilateral to the stroke is compared to a first predetermined amplitude threshold value. The absolute amplitude of the local maximum or minimum of the SEP ipsilateral to the stroke site above the first amplitude threshold value is indicative of a good outcome of the endovascular treatment. Alternatively or additionally, the absolute amplitude of the local maximum or minimum of the SEP ipsilateral to the stroke site below the first predetermined amplitude threshold value is indicative of a bad outcome of the endovascular treatment. Further alternatively, the latency of a local maximum or minimum of the SEP ipsilateral to the stroke is compared to a first predetermined latency threshold value, a latency below the threshold value indicating a good outcome.

Fig. 1

EP 4 223 223 A1

**Description**

**Technical field of the invention**

The present invention pertains to the field of medicine, and particularly, to the field of brain stroke. The invention relates to the use of a somatosensory evoked potential for an endovascular treatment as a marker and to a device thereof.

**Background of the invention**

Brain strokes are caused by a cerebral circulatory disorder that results in a temporary or permanent alteration of the functioning of one or more parts of the brain. These alterations generate focalized manifestations in motor functions such as paralysis of one half of the body (hemiplegia), facial paralysis and weakness in one limb; in sensitive functions such as loss of sight in one or both eyes and loss of balance; and in cognitive functions such as aphasia (loss of the capacity to produce or understand language) and disorientation, among others. In general, the symptoms of strokes are variable depending on the brain area affected. In milder cases, the consequences may go unnoticed or be confused with minor health issues such as headaches and may not be greatly limiting for the patient due to the anodyne nature of the symptoms. However, strokes frequently cause permanent neuronal damage or result in the death of the individual. Indeed, brain stroke is the second leading cause of mortality (5.5 million [95% UI 5.3-5.7], second only to ischemic heart disease) according to the 2016 Global Burden Disease Study. Stroke was also the second leading cause of global disability-adjusted life years (DALYs) (116.4 million [11.4-121.4]).

Strokes can be divided according to the nature of the lesion into cerebral ischemia and cerebral haemorrhage, with a proportion of 85% and 15%, respectively. An ischemic stroke appears when the brain loses blood supply due to the sudden and immediate interruption of blood flow, which frequently occurs due to the occlusion of any of the arteries that supply the brain matter because of a blood clot. The blood loss translates in a reduction of the glucose and oxygen flow to the brain, damaging the brain cells. In contrast, a haemorrhagic stroke appears when a brain blood vessel ruptures putting high pressure on brain cells, which damages them.

Acute ischemic strokes outcomes are highly dependent on the time taken to restore the normal circulation of blood in the brain as neurons are even more sensitive to ischemia than other cells in the body, due to their high ATP dependency and use. Therefore, the management of acute ischemic stroke is a major healthcare challenge. Over the last 15 years, advances in acute ischemic stroke management through specific protocols have led to a significant reduction in the morbidity and mortality related to this disease. Therefore, once the acute ischemic stroke diagnosis is confirmed by imaging techniques such as MR and/or CT upon arrival of the hospital, the etiological work up is conducted in parallel with therapeutic measures by stroke units.

Intravenous administration of tissue plasminogen activators (tPA's) have been widely used as a first line treatment in emergency ischemic stroke management for its clot breakdown capabilities. It has been demonstrated to improve survival and functional outcomes of patients (SITS-MOST study) when administered early from the ischemic stroke. However, its use is limited by the narrow therapeutic time window (nowadays the therapeutic window is <4.5 hours, although this time might be extended to < 6 hours, depending on the results of ongoing clinical trials) and by important contraindications, including coagulopathy, recent surgery, stroke or head injury within the past 3 months or being older than 80 years, among others. Also, it has an associated increase in intracranial haemorrhages. Ultimately, only a limited percentage of patients presenting with ischemic stroke can be eligible for treatment with intravenous tPA. Moreover, complete arterial recanalization with systemic thrombolysis, as well as recovery of functional independence, is achieved in less than 40% of patients, with this percentage clearly decreasing when the occlusion is of the proximal M1 segment of the middle cerebral artery (MCA) and the internal carotid artery (ICA).

These limitation of intravenous tPA have led to the search of complementary or alternative endovascular therapies for acute ischemic stroke, in particular mechanoaspiration and mechanical thrombectomy. Unlike intravenous tPA, endovascular treatments use mechanical devices applied by angiographically guided catheterisation to recanalize intracranial arteries occluded by clots. These have allowed to drastically reduce the duration of procedures, improve recanalization rates and clinical prognosis. Latest generations of endovascular therapies have demonstrated the superiority of these new devices with recanalization rates of over 68%.

Patient selection is usually made according to several clinical variables and neuroimaging parameters, as not all patients can benefit from endovascular interventions. Among the clinical variables, special attention is given to age, baseline NIHSS score (National Institute of Health Stroke Scale) -a tool used to objectively quantify the impairment caused by a stroke-, systolic blood pressure and hyperglycaemia. Regarding neuroimaging parameters, computed tomography (CT) and magnetic resonance imaging (MRI) have proved useful to provide crucial Information such as infarct core, ischemic penumbra/degree of collaterals, vessel occlusion, and thrombus that helps in the selection of the best candidates for endovascular treatment. However, achieving a complete arterial recanalization by means of endovascular treatments is not always sufficient to achieve an optimal clinical and functional recovery. Despite major advances in stroke therapeutics and using multivariate predictive factors based on clinical and neuroimaging criteria, there is still a 40-68% likelihood of significant disability or death after three months from stroke onset.

Therefore, it is clear that current patient selection parameters are still failing to properly capture the underlying processes that lead to a good or bad outcome of a stroke, and the outcome of endovascular intervention still cannot be reliably prognosticated. Early knowledge on the outcome of the endovascular intervention would provide valuable Information on the best therapeutic approach for the patient and to avoid unnecessary risks, such as intracranial haemorrhages. Therefore, there is still a need to more accurately predict the outcome of endovascular treatment on a patient prior to and after the treatment.

Known methods compare the affected brain hemisphere somatosensory evoked potential (SEPs) to the healthy cerebral hemisphere, but this has some drawbacks. First, there is a risk of error in the measurement of the SEP on both sides, which can lead to wrong decisions if the health brain hemisphere has low values, as a small change in the SEPs amplitude can lead to a high percentage difference between hemispheres. It is also limited to cases wherein the acute ischemic stroke is clearly lateral, this is only one hemisphere is affected, and therefore the results of the prediction rely on accurate analysis of the extension of the ischaemia. Given the importance of the time management of the outcome of the endovascular treatment this is a further problem these methods should address. There is therefore a need for a more reliable way to measure and accurately predict the outcome of endovascular treatment on a patient prior to and after the treatment that does not rely on the healthy cerebral hemisphere.

**Summary of the invention**

According to a first aspect of the invention, there is provided use of a somatosensory evoked potential (SEP), as a marker for predicting the outcome of an endovascular stroke treatment. The absolute amplitude of a local maximum or minimum of the SEP ipsilateral to the stroke is compared to a first predetermined amplitude threshold value and the absolute amplitude of the local maximum or minimum of the SEP ipsilateral to the stroke site above the first predetermined amplitude threshold value is indicative of a good outcome of the endovascular treatment and/or wherein the absolute amplitude of the local maximum or minimum of the SEP ipsilateral to the stroke site below the first predetermined amplitude threshold values is indicative of a bad outcome of the endovascular treatment. In some embodiments, the first predetermined amplitude threshold value defines a noise threshold such that the absolute amplitude of the local maximum or minimum of the SEP ipsilateral to the stroke site above the first predetermined amplitude threshold value indicates the presence of the SEP and/or the absolute amplitude of the local maximum or minimum of the SEP ipsilateral to the stroke site below the first predetermined amplitude threshold value indicates the absence of the SEP, optionally wherein the noise threshold is 0.1 $\mu V$.

In preferred embodiments, the SEP amplitude of the local maximum or minimum ipsilateral to the stroke site is compared to the first predetermined amplitude threshold value. A SEP amplitude of the local maximum or minimum ipsilateral to the stroke site above the first predetermined amplitude threshold value is indicative of a good outcome of the endovascular treatment. In some embodiments, the SEP is the N20 component and the input voltage is the lowest voltage at which the finger visibly twitches and the first predetermined amplitude threshold value is an absolute threshold value and is between 0.1 and 0.75 $\mu V$, preferably between 0.25 and 0.55 $\mu V$, more preferably between 0.3 and 0.4 $\mu V$.

In an embodiment, the use further comprises comparing the SEP amplitude of the local maximum or minimum ipsilateral to the stroke site to a second predetermined amplitude threshold value. A SEP amplitude of the local maximum or minimum ipsilateral to the stroke site below the second predetermined amplitude threshold value is indicative of a bad outcome of the endovascular treatment. In some embodiments, SEP is the N20 component and the input voltage is the lowest voltage at which the finger visibly twitches and the second predetermined amplitude threshold value is an absolute threshold value and is between 0.1 and 0.75 $\mu V$, preferably between 0.25 and 0.5 $\mu V$, more preferably between 0.3 and 0.4 $\mu V$.

In an alternative embodiment of the first aspect of the invention, there is provided use of a somatosensory evoked potential (SEP), as a marker for predicting the outcome of an endovascular treatment, The latency of a local maxima or minima of the SEP ipsilateral to the stroke is compared to a first predetermined latency threshold value, and the latency of a local maxima or minima of the SEP ipsilateral to the stroke site below the first predetermined latency threshold value is indicative of a good outcome of the endovascular treatment.

In some embodiments of the alternative embodiment of the first aspect of the invention, the use further comprises comparing the latency of a local maxima or minima of the SEP ipsilateral to the stroke to a second predetermined latency threshold value. The latency of a local maxima or minima of the SEP ipsilateral to the stroke site above the second predetermined latency threshold value is indicative of a bad outcome of the endovascular treatment.

The SEP may be determined by stimulating the median nerve or the ulnar nerve. The SEP may be selected from the N20 and P25 components; and preferably the SEP is N20.

The SEP may be determined by stimulating the tibial nerve. The SEP may be selected from the N35 and P40 components.

The amplitude or latency of a local maximum or minimum of the SEP may be combined with a quantitative assessment of the stroke severity or one or more clinical variables of the patient. In a further preferred embodiment, the quantitative assessment is the NIHSS and/or ASPECTS score and/or the collateral circulation state and/or the ischaemia core and/or TICI scale and/or wherein the clinical variables are selected from the age, the sex, the laterality of the stroke, the blood glucose levels and the mean arterial pressure of the patient.

According to a second aspect of the invention, there is provided an apparatus for predicting the outcome of an endovascular stroke treatment, comprising: a stimulator for providing electrical stimuli to one or more pairs of stimulating electrodes for stimulating a nerve; a voltmeter connected to one or more pairs of recording electrodes for recording an SEP resulting from the electrical stimuli provided to the nerve; and a processor configured to: compare the absolute amplitude of a local maximum or minimum of the SEP to a predetermined amplitude threshold value wherein the absolute amplitude of the local maximum or minimum of the SEP above the predetermined amplitude threshold value is indicative of a good outcome of the endovascular treatment and/or wherein the absolute amplitude of the local maximum or minimum of the SEP below the first predetermined amplitude threshold value is indicative of a bad outcome of the endovascular treatment; and/or compare the latency of a local maxima or minima of the SEP to a predetermined latency threshold value, wherein the latency of a local maxima or minima of the SEP ipsilateral to the stroke site below the first predetermined latency threshold value is indicative of a good outcome of the endovascular treatment.

**Brief description of the drawings**

To enable better understanding of the present disclosure, and to show how the same may be carried into effect, reference will now be made, by way of example only, to the accompanying schematic drawings, in which:

Figure 1A shows a stimulation apparatus for stimulating the patient with electrical pulses on the wrist of a patient;

Figure 1B shows a transcranial recording apparatus for measuring somatosensory evoked potentials placed on the scalp of the patient;

Figure 1C shows a voltage measured by the transcranial recording apparatus over time after stimulation of the median nerve;

Figure 2 shows a Tibial SEP recording defined by two major local extremes: negative (N35) and positive (P40) curvature waves.

Figure 3 shows the ROC curve and AUC value for different clinical variables of interest alone or in combination for the outcome prognostic prior to the MT at 7 days. A. NIHSS. B. N20 (pathological hemisphere log-transformed amplitude of N20). C. NIHSS + N20. D. NIHSS + ASPECTS. E. NIHSS + basal clinical variables (age, sex, laterality, blood glucose and mean arterial pressure (MAP)). F. NIHSS + basal clinical variables + N20.

Figure 4 shows the ROC curve and AUC value for different clinical variables of interest alone or in combination for the outcome prognostic prior to the MT at 90 days. A. N20 (pathological hemisphere log-transformed amplitude of N20). B. NIHSS + ASPECTS. C. NIHSS + baseline clinical variables (age, sex, laterality, blood glucose and mean arterial pressure (MAP)). D.

NIHSS + basal clinical variables + N20.

Figure 5 shows the ROC curve and AUC value for different clinical variables of interest alone or in combination for the outcome prognostic after the MT at 7 days. A TICI. B. N20 (pathological hemisphere log-transformed amplitude of N20) C. TICI + N20.

Figure 6 shows the ROC curve and AUC value for different clinical variables of interest alone or in combination for the outcome prognostic after the MT at 90 days. A TICI. B. N20 (pathological hemisphere log-transformed amplitude of N20) C. TICI + N20.

Figure 7 shows a schematic view of an apparatus according to one or more embodiments.

**Description of the invention**

**Definitions**

As used herein and in the appended claims, the singular forms "a", "an" and "the" include plural referents unless the context clearly dictates otherwise. Also, the use of "or" means "and/or" unless stated otherwise. Similarly, "comprise," "comprises," "comprising" "include," "includes," and "including" are interchangeable and not intended to be limiting.

Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods and compositions, the exemplary methods, devices and materials are described herein.

As used herein, the words or terms set forth below have the following definitions.

The term "modified Rankin scale" refers to a scale that measures degree of disability or dependence in the daily activities of people who have suffered a stroke or other causes of neurological disability.

The term "NIHSS" refers to the National Institutes of Health Stroke Scale, a systematic assessment tool that provides a quantitative measure of stroke-related neurologic deficit. The higher the values the more severe the stroke is.

The term "ASPECTS" refers to the Alberta stroke programme early CT score, a 10-point quantitative topographic CT scan score used for patients with stroke; where higher values indicate a lesser extent of ischaemia.

The term "TICI" refers to the Thrombolysis in Cerebral Infarction scale, a tool for determining the response of thrombolytic therapy for ischaemic stroke; where grade 0 indicates no perfusion and grade 3 complete perfusion.

The term "good prognosis" is preferably understood as the likelihood of a good outcome of the endovascular treatment. A good outcome is understood as a modified Rankin scale 0-2 at 90 days (after the endovascular therapy). The terms "prognosis" and "prediction" are used interchangeably in the present application.

The term "bad prognosis" is preferably understood as a modified Rankin scale ≥3 at 90 days after the stroke onset.

The term "dramatic recovery" is preferably understood as an improvement > 10 or NIHSS score 0-1 at 24 hours, as well as modified Rankin scale 0-2 at 90 days.

The term "potential" is preferably understood as the amplitude of a measured electrical signal, for example in the SEP signals context. Therefore, the term "amplitude" might be used alternatively.

The term "latency" is preferably understood as the time elapsed between a reference time and reception of a signal. The latency of a SEP may be absolute, understood as the time between the SEP stimulation and the recording of the SEP of interest at the scalp. The latency of a SEP may be relative to an expected time of the SEP after the stimulating signal is provided.

The term "CTP" is preferably understood as a perfusion computerised tomography, wherein a temporal sequence of images are taken between rinsing stages after the intravenous administration of an iodised contrast bolus to analyse the time density of the contrast and determine the perfusion of a certain area.

The term "Tmax" is preferably understood as the delay time between the start of the CT scanning and the detection of the maximum intensity of the contrast bolus. It is preferably expressed in seconds.

The term "ROC" is preferably understood as the receiving operating characteristic curve, which represents the diagnostic ability of a binary classifier system as its discrimination threshold is varied.

The term "AUC" is preferably understood as the area under (the ROC) curve, which represents the probability that a classifier will rank a randomly chosen positive instance higher than a randomly chosen negative one. An uninformative classifier would then yield an AUC of 0.5.

The term "quantitative SEP variable" is preferably understood as a SEP variable wherein the values are defined continuously in a range of values. The term "continuous SEP variable" may be used alternatively.

The term "dichotomic SEP variable" is preferably understood as a SEP variable wherein the values are defined discretely, typically as "present" or "absent". The term "categoric SEP variable" may be used alternatively.

**Description**

Evoked potential (EP) registering consists of stimulating a nerve and capturing the electrical response in the brain via electrodes placed on the scalp. Somatosensory EPs (SEP) consists of stimulating a sensory or mixed peripheral nerve and measuring the response by means of electrodes placed specifically on the scalp.

SEPs are a useful and non-invasive means of assessing the correct functioning of the somatosensory system. These evoked potentials are composed of a series of positive and negative deflections that are generated by the successive excitation of neural structures located along ascending somatosensory sensory tracts, for example the posterior cord pathway.

To generate a SEP, a stimulus capable of depolarising receptors that usually respond to different forms of mechanical stimulation (touch, vibration, tendon stretching...) must be applied, which may for example be achieved with electrical stimulation. They are mostly elicited by bipolar transcutaneous electrical stimulation applied on the skin over the trajectory of peripheral nerves of the upper limb (e.g., the median nerve) or lower limb (e.g., the posterior tibial nerve). Median nerve SEP may start with an 100-300 microsecond square or other wave electrical stimulus applied to the median or ulnar nerve at the wrist. The intensity (i.e. the amplitude of the electrical stimulus) is selected to cause visible thumb twitch, for example 1-2cm thumb twitch, without generating pain to the patient. The electrical stimulus generates a depolarization cascade along the nerve until it reaches the brain. Along its path, several neurons and nerve ganglions are involved generating the positive ($P_x$) and negative ($N_x$) deflections on the potential (the x value refers to the latency of such deflection measured in ms from the stimulation time). Px SEPs are seen as local minima of the SEP amplitude and $N_x$ SEPs are seen as local maxima of the SEPs amplitude. The response is measurable by means of electrodes placed on the scalp.

In upper limb SEP, upon delivery of such a stimulus, nerve action potentials travel up sensory fibres to the shoulder, producing a minimum in the amplitude as they enter the spinal cord from the brachial plexus around vertebra C7. This local minimum is formally known as $P_9$. In the course of conduction, the sensory fibres then transverse the cervical roots and enter the cervical cord. The median nerve pathway then joins the posterior columns, sending off collateral branches to synapse in the mid-cervical cord. This mid-cervical cord activity gives rise to a local minimum in the amplitude known as $P_{13}$. Further conduction in the posterior columns passes through the synapse at the cervico-medullary junction and enters the decussation of the lemnisci. A scalp $P_{14}$ amplitude local minimum is generated at this level. As conduction continues up the medial lemniscus to upper midbrain and into the thalamus, a scalp local maximum is detected, the $N_{18}$. After synapsing in the thalamus and traversing the internal capsule, the amplitude local maximum $N_{20}$ is recorded over the somatosensory cortex (parietal region) contralateral to the wrist stimulated, corresponding to arrival of the nerve impulses at the primary somatosensory region. The generators of the components following $N_{20}$ such as $P_{25}$ are not known in detail but are thought to be cortical structures receiving afferents from primary somatosensory cortex, such as secondary somatosensory cortex and cortical associative areas. Accordingly, the $N_{20}$ and the $P_{25}$ response can be recorded by one or more measuring electrodes placed on the scalp near the parietal region. It is best recorded using an electrode placed 3-4 cm posterior to C3 or C4.

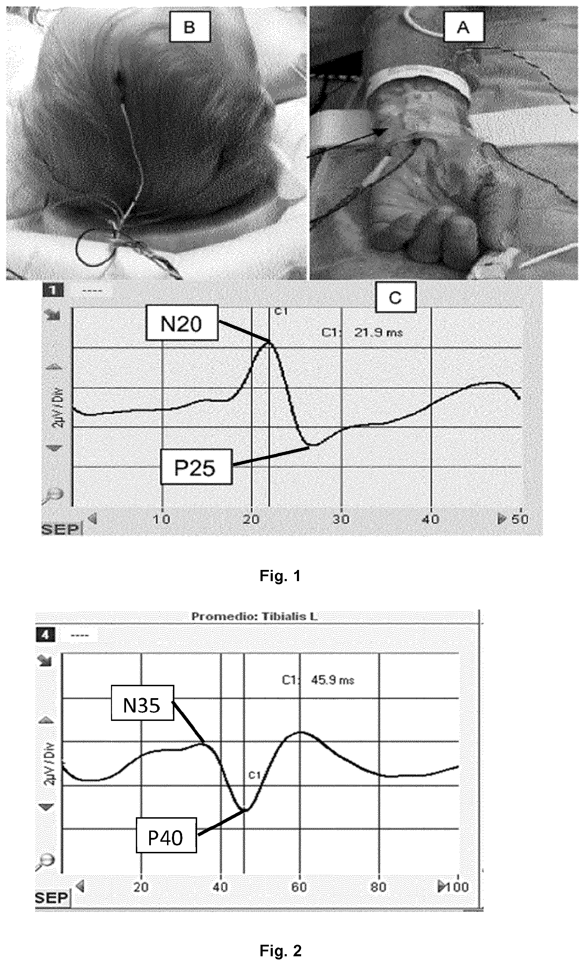

Figure 1 shows an example of a median nerve SEP recording setup and an upper limb SEP recording. Figure 1A shows the electrode placement in the wrist for the electrical stimulation of the ulnar nerve on the upper limb. Figure 1B shows the electrode placement in the scalp to record the SEP. Figure 1C depicts the SEP potential recorded by the electrodes placed on the scalp after stimulation from the electrode as shown in Figure 1B. The potential amplitude is showed over time, with the stimulation time as the reference time t=0ms. SEP $N_{20}$ can be seen as a peak with negative curvature (i.e. as a local maximum) in the potential on the recorded signal 20 ms after the stimulation is performed. In the particular example of Figure 1C, the SEP $N_{20}$ appears 22ms after the stimulation is performed. Similarly, SEP $P_{25}$ can be seen as a peak with positive curvature (i.e. as a local minimum) in the potential on the recorded signal 25 ms after the stimulation. In the particular example of Figure 1C, the SEP $N_{20}$ appears 26 ms after the stimulation is performed. The small difference in the latency for each SEP with respect to the reference time respond to physiological factors as will we further explained below. The SEP amplitude it is therefore understood as the potential local maximum or minimum value recorded from the scalp for a determined SEP event.

It must be noted that physiological differences between subjects, like height, affect the latency of the SEPs. Therefore, the peripherical differences can be eliminated by adjusting the central conducting time (CCT) defined as the interval between the $P_{14-16}$ and the $N_{20}$ amplitude local extrema. Measurements of the $P_{14-16}$ can be taken at a cervical level (e.g. spinal C6) and at a transcranial location. The CCT is defined as the interval between the time nerve conduction enters at the spinal level and the start of the $N_{20}$ response. The differences can also be eliminated by measuring the latency difference between a given transcranial recorded SEP (such as the $N_{20}$) and evoked potentials at Erb's point. The latency difference can be subtracted from the measured absolute SEP latency value to account for peripheral abnormalities in conduction of the electrical signal to the brain.

In the lower limbs, a similar reasoning of the potential travelling up the posterior tibial nerve gives rise to the different deflections on the potential. The evoked potential after electrical stimulation of the posterior tibial nerve has as its main component a local maximum with a latency of approximately 35 ms ($N_{35}$) followed by a large local minimum at a latency of 40 ms ($P_{40}$), which is assumed to originate from cortical structures. N35 and P40 are best recorded using an electrode 3-4 cm posterior to Cz with a frontal or cephalic reference placed at high cervical level. The response generated in the popliteal fossa following tibial nerve stimulation is equivalent to that evoked at Erb's point in upper extremity SEPs. It determines whether the tibial nerve has been appropriately stimulated and can distinguish whether an increase in absolute latencies is due to a peripheral problem such as polyneuropathy.

Figure 2 shows an example of a lower limb SEP recording. The potential change is showed over time, with the stimulation time as the reference time t=0ms. SEP $N_{35}$ can be seen as local maximum in the potential on the recorded signal 35 ms after the stimulation is performed. Similarly, SEP $P_{40}$ can be seen as a local minimum in the potential on the recorded signal 45 ms after the stimulation.

To obtain a response ipsilateral to the stroke site, it is necessary to stimulate a nerve, such as the median and/or ulnar nerve, contralateral to the stroke site; and vice versa. In some embodiments, both side nerves are stimulated to assess the SEP responses on the sides of the brain ipsilateral and contralateral to the stroke site. Therefore, the healthy SEP response is obtained from the ipsilateral median, ulnar or tibial nerve while the impaired SEP response is obtained from the contralateral median, ulnar or tibial nerve.

A first aspect of the invention refers to the use of a SEP as a marker for predicting the outcome of an endovascular stroke treatment. Data quantitatively indicating the SEP amplitude of the patient ipsilateral to the stroke site may be received. The SEP is then used as follows: the absolute amplitude of a local maximum or minimum of the SEP ipsilateral to the stroke is compared to a first predetermined amplitude threshold value wherein the absolute amplitude of the local maximum or minimum of the SEP ipsilateral to the stroke site above the first predetermined amplitude threshold value is indicative of a good outcome of the endovascular treatment and/or wherein the absolute amplitude of the local maximum or minimum of the SEP ipsilateral to the stroke site below the first predetermined amplitude threshold values is indicative of a bad outcome of the endovascular treatment.

The endovascular treatment may be planned, or ruled out, based on the observed value of the SEP.

It has been found that a patient's SEP ipsilateral to the stroke site, before, during and after endovascular treatment is performed, is a good indicator of whether the endovascular treatment will have a good and/or bad outcome for the patient (see Example).

SEPs are defined by a specific latency (x) since the moment the potential is elicited, and a determined local extremum, either a local maximum or a local minimum. SEPs with a negative curvature ($N_x$), are local maxima while SEPs with a positive curvature ($P_x$) are local minima. However, in real world conditions the latency associated to the neurological process of interest (for example the arrival of the nerve impulses at the primary somatosensory region for N20) might not have the expected latency in certain patients, due to anatomical differences in their neural paths. It is therefore defined a $\pm$5ms range wherein a determined SEP might be found.

For example, it is considered that N20 can be recorded between 15 and 25ms after the potential is triggered.

The SEP is a non-invasive marker that can be applied quickly to a patient. The use of a non-invasive marker to determine the endovascular treatment outcome of a patient suffering from acute ischemic stroke is vital in the stroke units, as it can effectively help to assign resources in a time-constraint driven field, and avoid the unnecessary risks deriving from invasive interventions to the patient. It is noted that the recording of SEPs can be achieved in short time from the hospital arrival and therefore these decisions can be taken as soon as possible, before any neuroimage technique that can provide an ASPECTS score to assess the extent of early ischaemic changes in the middle cerebral artery territory. Furthermore, the SEP could be recorded in a pre-hospital environment (e.g. in an ambulance) so the patient could be assessed before the arrival at the hospital, with all the timing benefits associated from this quick assessment. In other words, the SEP values (presence/absence, amplitude or latency) can be determined rapidly in real-time in a non-invasive manner, and the use of SEPs provides a more accurate indication regarding the brain functional status than currently-used clinical factors, allowing staff to make accurate and fast decisions in both a pre- and in-hospital setting.

In some embodiments, the first predetermined amplitude threshold value defines a noise threshold such that the absolute amplitude of the local maximum or minimum of the SEP ipsilateral to the stroke site above the first predetermined amplitude threshold value indicates the presence of the SEP, and/or the absolute amplitude of the local maximum or minimum of the SEP ipsilateral to the stroke site below the first predetermined amplitude threshold value indicates the absence of the SEP. In these embodiments, the noise threshold is determined by the value of the noise in the signal, wherein the noise is defined as all signal values that do not represent a SEP but are derived from the electrical noise in the signal and/or the recording of the signal and/or the processing of the signal. In a more preferred embodiment, the noise threshold is 0.1 $\mu$V.

In some embodiments, the SEP amplitude of the local maximum or minimum ipsilateral to the stroke site is compared to the first predetermined amplitude threshold value. A SEP amplitude of the local maximum or minimum ipsilateral to the stroke site above the first predetermined amplitude threshold value is indicative of a good outcome of the endovascular treatment. The value for the first predetermined amplitude threshold value depends on the input voltage applied to the patient, and position and type of measuring electrodes used. However, the first amplitude threshold value is preferably determined by measuring the "healthy" SEP responses in one or more patients who are considered to have healthy brain function when a spam is visible in the SEP eliciting point (e.g. fingers or toes visibly twitching), and selecting a threshold potential value for the setup which corresponds to the first predetermined amplitude threshold, as shown in the Example. It is noted that the SEP amplitude and the first predetermined amplitude threshold value may be an absolute amplitude value the logarithm may be taken so that the logarithmic amplitude value is taken.

In a preferred embodiment, wherein the SEP is the N20 component and the input voltage is the lowest voltage at which the finger visibly twitches, the first predetermined amplitude threshold value is an absolute threshold value and is between 0.1 and 0.75 $\mu$V, preferably between 0.25 and 0.5 $\mu$V, more preferably between 0.3 and 0.4 $\mu$V.

In some embodiments, the use of the SEP comprises comparing the SEP amplitude of the local maximum or minimum ipsilateral to the stroke site to a second predetermined amplitude threshold value. A SEP amplitude of the local maximum or minimum ipsilateral to the stroke site below the second predetermined amplitude threshold value is indicative of a bad outcome of the endovascular treatment. The value for the second predetermined amplitude threshold value depends on the input voltage applied to the patient, and position and type of measuring electrodes used. However, the second amplitude threshold value is preferably determined by measuring the "unhealthy" SEP responses in one or more patients who are considered to have unhealthy brain function when a spasm is visible in the SEP eliciting point (e.g. fingers or toes visibly twitching), and selecting a threshold potential value for the setup which corresponds to the second predetermined amplitude threshold. It is noted that the SEP amplitude and the first predetermined amplitude threshold value may be an absolute amplitude value the logarithm may be taken so that the logarithmic amplitude value is taken.

In a preferred embodiment, , wherein the SEP is the N20 component and the input voltage is the lowest voltage at which the finger visibly twitches, the second predetermined amplitude value is an absolute threshold value and is between 0.1 and 0.75 $\mu$V, preferably between 0.25 and 0.5 $\mu$V, more preferably between 0.3 and 0.4 $\mu$V.

It is noted that the first and second predetermined amplitude threshold values can be used in combination or alone. Therefore, the SEP amplitude value can be a marker for indicating that the endovascular treatment will have a good outcome (if it is above the first predetermined amplitude threshold value), or a marker for indicating that the endovascular treatment will have a bad outcome (if it is below the second predetermined amplitude threshold value) or a marker of both, where being above the first predetermined amplitude threshold value is a marker for good outcome and being below the second predetermined amplitude threshold value is a marker for bad outcome. It is also noted that the first and second predetermined amplitude threshold values may or may not be the same value.

An alternative embodiment of the first aspect of the invention, refers to the use of a SEP as a marker for predicting the outcome of the endovascular treatment. Data quantitatively indicating the SEP latency of the patient ipsilateral to the stroke site may be received. The SEP is then used as follows: the latency of a local maxima or minima of the SEP ipsilateral to the stroke is compared to a first predetermined latency threshold value wherein a SEP amplitude ipsilateral to the stroke site below the first predetermined latency threshold value is indicative of a good outcome of the endovascular treatment.

As latency is dependent on the pathway length, strokes tend to provoke longer pathways for the SEPs, as the direct pathway is either not available or the neurons involved are less responsive. Therefore, having a latency below the first predetermined latency value indicates that the ipsilateral site of the stroke is less damaged, or at least closer to a normal functioning pathway, and can be used as a marker of a good outcome of the endovascular treatment as an alternative to measuring the SEP amplitude.

It is noted that latency value and threshold may be modified to avoid the "0" value effect. This effect arises from the patients with an absent SEP, which may be assigned a null or zero latency. As the latency is inversely correlated with the good outcome probability, paradoxically these patients are expected to have the best outcome by the regression algorithms. In some embodiments, the latency registered signal is transformed by correcting its value to the absolute difference with the expected latency, i.e.

$$L = Abs(L_0 - x),$$

wherein L is the modified latency, $L_0$ the original measured latency and x the expected latency for a given SEP. By this way, patients with a 0 latency are actually given an x latency, which will be bigger than any of those with a good (low) latency.

In some embodiments, the use further comprises comparing the latency of a local maxima or minima of the SEP ipsilateral to the stroke to a second predetermined latency value, wherein the latency of a local maxima or minima of the SEP ipsilateral to the stroke site above the second predetermined latency threshold value is indicative of a bad outcome of the endovascular treatment. In a similar reasoning as before, having a latency above the second predetermined latency threshold value indicates that the ipsilateral site of the stroke is more damaged, or at least further from a normal functioning pathway, and can be used as a marker of a bad outcome of the endovascular treatment.

It is noted that any of the preferred embodiments may use the first and second predetermined latency threshold value in combination or alone. Therefore, the latency of a local maxima or minima of the SEP can be a marker for indicating that the endovascular treatment will have a good outcome (if it is below the first predetermined latency threshold value), or a marker for indicating that the endovascular treatment will have a bad outcome (if it is above the second predetermined latency threshold value) or a marker of both, where being below the first predetermined latency threshold value is a marker for good outcome and being above the second predetermined latency threshold value is a marker for bad outcome. It is also noted that any of the preferred embodiments may use the first and second predetermined latency threshold values in combination or alone.

In some embodiments, the SEP is determined by stimulating the median nerve and/or the ulnar nerve. In some embodiments, the SEP is selected from the $N_{20}$ and $P_{25}$ components; preferably wherein the SEP is $N_{20}$. The $N_{20}$ and

$P_{25}$ components are local extrema components of the SEP recordings that occur with 20 and 25 ms latency from the input, respectively. The $N_{20}$ is a local maximum peak while the $P_{25}$ is a local minimum peak.

In some embodiments, the SEP is determined by stimulating the tibial nerve. In some embodiments, the SEP is selected from the $N_{35}$ and $P_{40}$ components. The $N_{35}$ and $P_{40}$ components are peak components of the SEP recordings that occur with 35 and 40 ms latency from the input respectively. The $N_{35}$ is a local maximum peak while the $P_{40}$ is a local minimum peak.

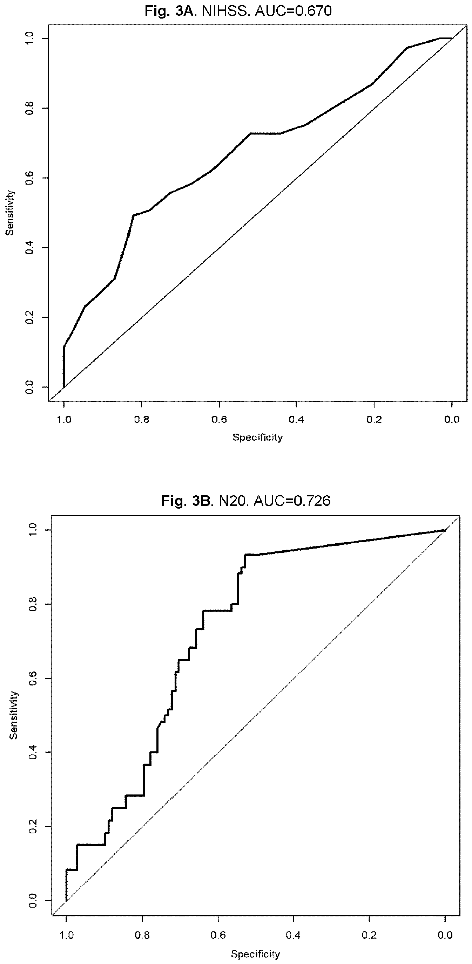

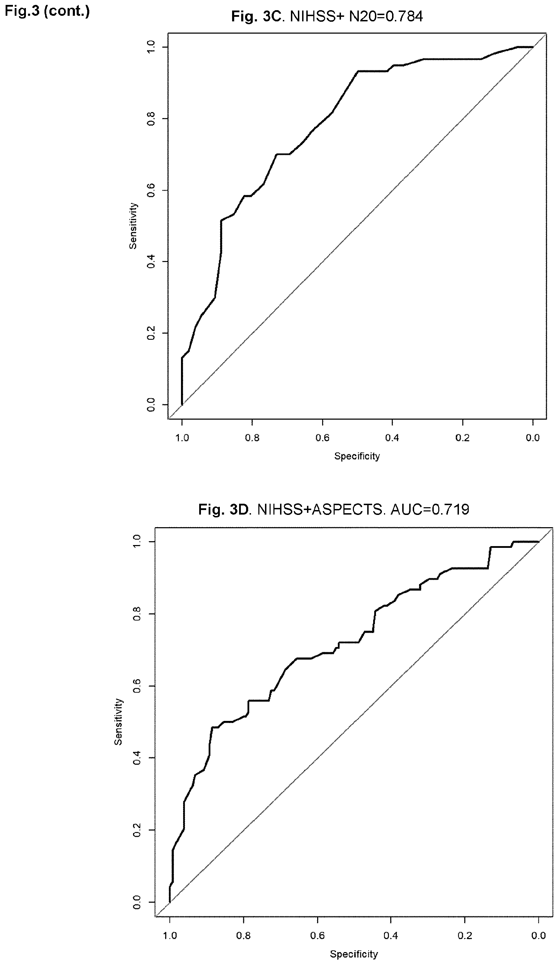

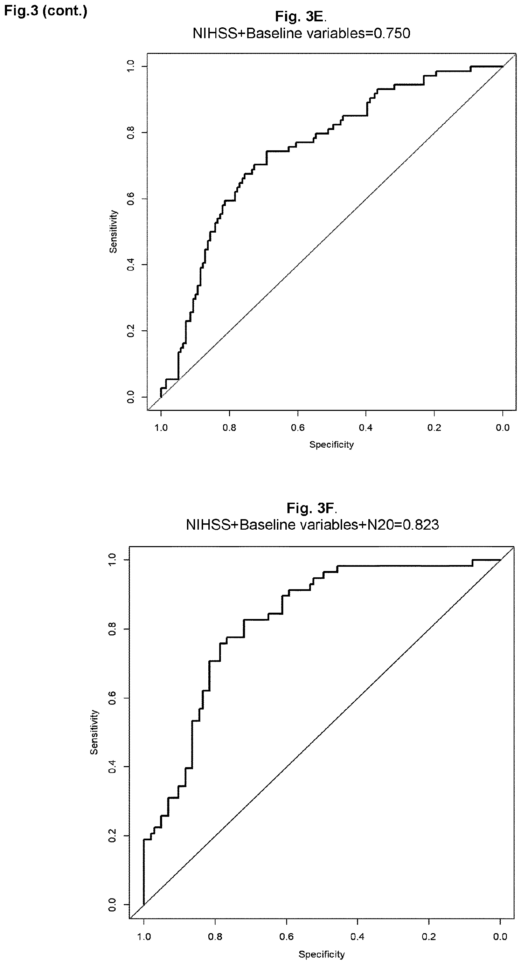

In some embodiments, the amplitude and/or latency of a local maximum or minimum of the SEP is combined with a quantitative assessment of the stroke severity or one or more clinical variables of the patient. While the SEP has a predictive power by itself, it can be further combined with current outcome of the endovascular treatment prediction systems which generally involves a quantitative assessment of the stroke severity and using one or more clinical variables of the patient; so that the prediction is more accurate (see Figure 3).

In some embodiments, the quantitative assessment is the NIHSS and/or ASPECTS score and/or the collateral circulation state and/or the ischaemia core and/or TICI scale; . The clinical variables might be, although are not limited to: the age, sex, laterality of the stroke, the blood glucose levels and the mean arterial pressure (MAP) levels. It is noted that although using the SEP alone has a better predictive capability of a good outcome prognosis at 7 days post-stroke than the NIHSS scale and the combination of the NIHSS and the ASPECTS scores, the combination of the NIHSS and N20 variables and NIHSS, baseline variables and $N_{20}$ have a higher predictive ability than the NIHSS scale alone and the combination of NIHSS and ASPECTS (see Figures 3-6).

It has been shown that N20 adds predictive power to the clinical variables currently used (NIHSS, ASPECTS) when the log- transformed N20 values were further used in a logistic regression model for good functional prognostic prediction. The effect has been shown to be slightly more evident in the prognosis at 90 days than at 7 days (see Example Section 2.7).

It is noted that the use of a SEP as a marker for predicting the outcome of an endovascular stroke treatment according to any of the embodiments herein described may be performed before, during or after the endovascular stroke treatment is performed.

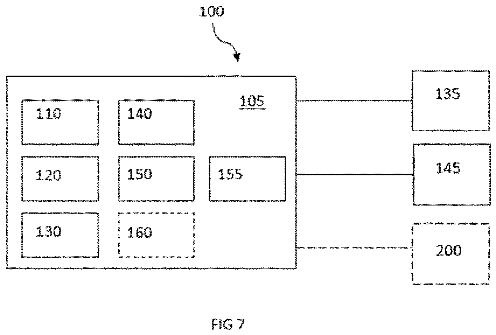

Fig. 7 shows a schematic view of an apparatus 100 for predicting the outcome of an endovascular stroke treatment through measurement of a somatosensory evoked potential (SEP). The apparatus 100 comprises SEP module 105 comprising processor 110, memory 120, stimulator 130, voltmeter 140, timer 150 and user interface 155. The SEP module 105 is electrically connected to one or more pairs of stimulating electrodes 135 and one or more pairs of recording electrodes 145. The SEP module 105 may additionally comprise a communications module 160. The communications module 160 may be communicatively connected (i.e. wired or wirelessly) to an external display device 200 for displaying information recorded by the apparatus 100. Alternatively, the SEP module 105 may comprise a display device incorporated into the module.

The memory 120 stores instructions for the processor 110 for a SEP measurement procedure. The procedure may be configured to measure any SEP, but preferably is configured to measure the N20 SEP. The instructions may be comprised in a computer program product implemented in hardware and/or software. In particular, during the SEP measurement procedure, the instructions instruct the processor 110 to control the stimulator 130 in order to provide a stimulating electrical potential between the one or more pairs of stimulating electrodes 135. The memory 120 further stores instructions for the processor 110 to control the timer 150, and in particular initiate the timer 150 when the stimulator 130 provides the stimulating electrical potential. The memory 120 is further stores instructions for the processor 110 to measure the voltage measured by the voltmeter 140 at a plurality of time instances after the stimulating electrical potential is provided, and to measure the time recorded by the timer 150 for each voltage measurement. The memory may comprise one or more volatile or nonvolatile memory devices, such as DRAM, SRAM, flash memory, read-only memory, ferroelectric RAM, hard disk drives, floppy disks, magnetic tape, optical discs, or similar, and may be provided in the SEP module 105 or partially or wholly external to the SEP module 105 and communicatively connected to the SEP module 105.

The processor 110 is configured to control the stimulator 130, timer 150, and voltmeter 140. The processor 110 is further configured to follow the instructions for the SEP measurement procedure when an indication from the user interface 155 is received indicating that the procedure should begin. The processor 110 may comprise one or more processing units, such as a microprocessor, GPU, CPU, multi-core processor or similar, and may be internal or external to the SEP module 105 or distributed between processors of different devices.

The stimulator 130 is electrically connected to the one or more pairs of stimulating electrodes 135 and configured to provide one or more electrical stimuli to the one or more pairs of stimulating electrodes 135 (i.e. to provide a potential between the one or more pairs of electrodes). The electrical stimuli comprises one or more electrical pulses. The pulses may comprise, for example, a square or sine wave pulse and may be, for example, less than 1ms in duration, preferably between 0.1 ms and 0.3 ms in duration and more preferably 0.2 ms in duration. The electrical stimuli may be provided at any suitable frequency, for example between 1 Hz and 10 Hz, and more preferably between 5 Hz and 6 Hz. The stimulator 130 is configured to provide an electrical stimulus of sufficient intensity such that visible twitching of the finger or toe on which the one or more pairs of stimulating electrodes 135 are placed occurs. In some embodiments, the stimulator 130 may be configured to apply electrical stimuli of a fixed, predetermined intensity which is determined to provide the correct amount of stimulation to the finger or toe. In other embodiments, the stimulator 130 may be controlled by the processor 110 to provide stimuli over a range of predetermined electrical intensities. For example, a user may select an electrical intensity using the user interface 155, and the processor 110 may control the stimulator 130 to provide the electrical stimuli at the selected electrical intensity. The stimulator 130 may be configured to provide electrical stimuli having a current intensity of between 0 mA and 50mA.

The voltmeter 140 is connected between the one or more pairs of recording electrodes 145 and is configured to measure the electrical potential between the recording electrodes 145. The voltmeter may be any suitable voltmeter known in the art. The voltmeter 140 preferably has a time resolution of 5ms or less, preferably 1ms or less and more preferably 0.1ms or less, such that the amplitude peak of the SEP can be accurately determined. The voltage resolution of the voltmeter is preferably $0.1\mu V$ or less.

The components of the SEP module 100 may be powered by an external power source via a power connection (not shown), or may comprise an internal power source such as a battery. The power source also may be configured to power the display device 200 or the display device 200 may be powered by a separate external power source.

The timer 150 may be any suitable timer. The timer preferably has a time resolution at least as short as the time resolution of the voltmeter 140 such that the voltage measurements can be accurately timed. The timer 150 may be included in the voltmeter 140 or provided separately. The one or more pairs of stimulating electrodes 135 are configured to be placed on the skin of a user such that an exposed surface of each of the electrodes contacts the skin of the user. The one or more pairs of stimulating electrodes 135 may be provided on a substrate with an adhesive surface configured to removably attach to the skin of the user. The one or more pairs of stimulating electrodes 135 may be provided on the interior surface of a glove or sock in a location corresponding to the position of the desired nerve (e.g. the median or ulnar nerve) to be stimulated.

The one or more pairs of recording electrodes 145 are configured to be placed on the scalp of a user such that an exposed surface of each of the electrodes contacts the skin of the scalp of the user. The one or more pairs of stimulating electrodes 135 may be provided on a substrate with an adhesive surface configured to removably attach to the skin of the user. The one or more pairs of recording electrodes 145 may be provided on the interior surface of a cap in a location of the cap corresponding to the desired location on the scalp to record. In particular, the location of the one or more pairs of recording electrodes 145 may be provided at a location of the cap proximal to the bilateral somatosensory cortex, and more specifically the C3', C4' and/or Cz' positions.

The user interface 155 may be any suitable user interface for providing user input to the SEP module. The user interface may for example be a touch screen or other display device with guided user interface (GUI); or may comprise one or more buttons, switches, levers and the like. In some embodiments the user interface 155 is also the display device 200. The user may select various variables via the user interface 155 for the SEP measurement procedure. For example, the user may select the electrical intensity of the electrical stimuli, start and stop the measurement procedure, select how many electrical stimuli are provided during the procedure and so on.

Display device 200 may be any suitable display device for displaying the results of the SEP measurement procedure or other information. The display device 200 may for example be a mobile device, tablet, computer or screen. The display device 200 may include user interface 155 (e.g. a GUI) for controlling the SEP module 105.

The apparatus 100 may be used as follows for an SEP measurement procedure. Firstly, a user may place the one or more pairs of stimulating electrodes 135 on the desired location on a patient. For example, the stimulating electrodes 135 may be placed proximal to a nerve which causes the finger or toe of the patient to twitch when stimulated (e.g. the median or ulnar nerve). The one or more pairs of recording electrodes 145 may be placed on the desired located on the patient's head in order to record the SEP. For example, the user may place the one or more pairs of recording electrodes 145 on the C3', C4' and/or Cz' locations for recording N20 SEPs. The SEP module 105 may then be initiated. In some embodiments, the apparatus 100 may comprise a impedance or resistance meter (not shown) configured to measure the impedance between the one or more pairs of recording electrodes 145. The apparatus 100 may be configured to measure the impedance before starting the SEP measurement procedure and alert the user (for example via user interface 155 or display device 200) that the impedance between the one or more pairs of recording electrodes 145 is not below a predetermined impedance threshold. The apparatus 100 may be configured to start the SEP measurement procedure when the measured impedance falls below the predetermined impedance threshold indicating that the pairs of electrodes all correcting contact the skin of the patient. In some embodiments, the user may use the SEP module 105 in a preliminary procedure to select the correct electrical intensity for the electrical stimulus, and in particular the lowest electrical intensity which causes a visible twitch of the patient's finger or toe to occur. The user may adjust the electrical intensity by the user interface 155. In other embodiments, the preliminary procedure may not occur, and a preselected electrical intensity may be used in the SEP measurement procedure. The user may select other parameters for the procedure such as the number of pulses via the user interface 155, and initialise the measurement procedure via the user interface 155. The SEP module 105 then provides the one or more electrical stimuli via the stimulator 130, and records the response measured at the one or more pairs of recording electrodes 145 as a function of time. The response may be saved in the memory 120. The processor 110 is configured to calculate the local maximum or minimum voltage amplitude associated with the measured SEP potential measured by the voltmeter (i.e. to determine whether the local maximum or minimum is present within an expected time window associated with the SEP, for example the expected time instance of an SEP +/- 5ms). Where multiple electrical stimuli are provided, the processor 110 may calculate the average maximum or minimum voltage amplitude of the SEPs. The maximum or minimum may be calculated by any suitable method such as interpolation of the measured values or a best-fit algorithm. Alternatively, the measured potential values as a function of time may be displayed on display device 200 and the user may manually input the amplitude value for the maximum or minimum (and optionally the latency) via user interface 155.

In some embodiments, the processor 110 is configured to compare the amplitude of the local maximum or minimum with a predetermined threshold value saved in memory 120. The predetermined threshold value in some embodiments may define a noise threshold such that a maximum or minimum with an absolute value greater than the noise threshold indicates the presence of the measured SEP. For example, the noise threshold may be determined by determining the maximum or average voltmeter measurement when no electrical stimuli is applied, such that voltage measurements of that order of magnitude are associated with noise present in the system and not with any measured SEP signal. In some embodiments, the noise threshold may be 0.1 $\mu$V. The predetermined threshold value may be another threshold value with an optimal specificity and sensitivity as disclosed herein.

In some embodiments, the processor 110 is configured to determine the latency of the maximum or minimum associated with the measured SEP potential measured by the voltmeter. The latency may be relative to the time of electrical stimulus provided by the stimulating electrodes or relative to the expected time of the maximum or minimum after the electrical stimuli is provided. The latency may be compared to a predetermined latency threshold value saved in memory 120. Where multiple electrical stimuli are provided, the processor 110 may calculate the average latency of the SEP maxima or minima.

The processor 110 may be configured to output information regarding the SEP measurement procedure to display device 200. The information may include one or more of the measured voltage amplitude of the maximum or minimum associated with the SEP and the measured latency of the maximum or minimum. The information may include whether the amplitude and/or the latency is above or below a threshold value. The information may include an indication of whether a good or bad patient outcome is predicted for the endovascular treatment. The information may include a probability value of optimal functional recovery of the patient from the endovascular treatment. Display device 200 displays the information to the user.

In some embodiments, the memory 120 may include other clinical variables associated with the patient including a quantitative assessment of the stroke severity of the patient. Alternatively, the user may input these clinical variables via user interface 155. The variables may include the NIHSS score, ASPECTS score, age, sex, laterality of the stroke, blood glucose levels and mean arterial pressure of the patient. The processor 110 may predict the outcome of the endovascular treatment using the measured amplitude or latency of the measured SEP maximum or minimum in combination with one or more clinical variables, using a combined model saved in the memory 120.

The apparatus 100 may be used to implemented any of the uses or methods disclosed herein.

**Example**

**1. Methodology**

Patients: eligible patients were between 18 and 90 years of age, with an occlusion of the anterior circulation (M1 or M2 segment of the middle cerebral artery with or without a significant stenosis or occlusion of the ipsilateral internal carotid artery diagnosed by CT-angiography (CTA) or MR-angiography, according to the data available from experimental models. They were treated within the first 8 hours after symptom onset or after the last hour of asymptomatic onset -in patients with an awakening stroke or a stroke of uncertain chronology-, with functional independence prior to stroke, as measured by a modified Rankin scale score of less than or equal to 2 (where 0 means no symptoms and 6 indicating dead) and with a baseline in the National Institutes of Health Stroke Scale (NIHSS) score of 6 or higher, ranging from 0 to 42 (the higher the score, the more severe the stroke). The main imaging exclusion criterion is evidence of extensive ischaemic core. Therefore, patients with a score equal to or less than 5 on the Alberta Stroke Program Early Computed Tomography Score (ASPECTS) on plain CT or less than or equal to 4 on the MR diffusion sequence were excluded from the study. These core cut-off points have been selected according to the treatment effect of mechanical thrombectomy shown in the HERMES collaborative group meta-analysis (presented at the International Stroke Conference, Houston 2017). Furthermore, patients with a well-documented history of neuromuscular disease, stroke or nervous system tumours that may interfere with the assessment of PES were also excluded.

Clinical and neuroimaging data: Clinical and anthropometrical variables were registered: age, sex, location of intracranial occlusion, type of treatment and the presence of cardiovascular risk factors as arterial hypertension, Diabetes Mellitus, dyslipidaemia and obesity. Severity of the stroke was evaluated by the NIHSS at the time of admission. Time variables from stroke onset to the different diagnostic and treatment interventions during the acute phase of stroke were also recorded.

Baseline neuroimaging can be either non-contrast head CT (NCCT) together with angio-CT (CTA), multiparametric MRI (MRI) or perfusion CT (CTP) if MRI is not available. Control non-contrast head computed tomography (NCCT) or MRI was performed at 24 hours to determine infarct volume and to discard intracranial haemorrhage or malignant oedema. Angio-CT (CTA), angio-MRI (MRA) or transcranial duplex (TCD) were used to confirm persistence of revascularisation at 24 hours. The variables analysed in the control neuroimages were the volume and location of the ischaemic lesion, the presence of haemorrhagic transformation and the arterial status.

The CTP protocol was performed on a 64-detector scanner (APV General Electrics, GE Medical Systems; Milwaukee, Wisconsin, USA) and included CBF, CBV, MTT and perfusion mapping (TMax). Tissue volume with a delay in TMax>2s, TMax>4s, TMax>6s and TMax>8s was assessed to establish a gradient of hypoperfusion severity. Tissue with a delay in TMax>6s was considered as tissue at ischaemic risk.

The MRI protocol was performed with Siemens Magneton Verio 3 Tesla equipment with a 32-channel magnet. The acquired sequences were axial slices SWI/gradient echo (GRE), DWI, FLAIR (attenuation inversion recovery imaging), Angio-MR TOF, dMRA (dynamic postcontrast MR angiography) and perfusion study, which was performed immediately after the dynamic study.

All images were anonymised and stored in DICOM format. Investigators blinded to clinical prognosis and SEP monitoring assessed all neuroimaging studies. They performed a visual analysis of qualitative variables and a quantitative analysis of post-processed variables: first-pass brain perfusion (DSC: Dynamic Susceptibility Contrast) and perfusion maps. Post-processing of DWI and perfusion sequences was performed with the Olea Sphere platform. In addition, RAPID software (iSchemaView, Redwood City, CA) was used to calculate baseline and control ischaemic lesion volume in MRI DWI sequences and rCBF maps and for automatic evaluation of the ASPECTS scale in the NCCT and MRI DWI sequence. Pre-procedural imaging variables include the volume and location of the ischaemic core and baseline hypoperfused tissue, the severity of hypoperfusion, the location of the occlusion and the status of the collateral circulation according to the Arterial Collateral Gradind Scale (ACG) on angio-CT or the ASITN/SIR Collateral Grading System scale if the neuroimaging performed was a dMRI.

Conventional angiography assessed the location of arterial occlusion and the degree of recanalization according to the modified TICI criteria, which define complete recanalization as TICI 2b, 2c and 3. The time of femoral puncture (GP), the number of stentriever passes necessary to achieve revascularisation and the time of arterial recanalization was recorded. Baseline blood pressure and blood glucose and, subsequently, blood pressure was collected periodically (every 5 minutes). Conscious sedation was used whenever possible.

EP monitoring: evoked potentials were recorded using the 10-channel electromyographic system Medelec Synergy™ (Vyasys Healthcare) and the intraoperative monitoring system ISIS (Inomed Medizintechnik GmbH).

Although both MEP and SEP monitoring were initially planned, the acute ischemic stroke protocol of the centre required the patient to be awake or semi-anesthetised which makes MEP monitoring painful. This prevents the patient to collaborate by being in strict rest during the endovascular procedure. Therefore, only SEPs were monitored.

Stimulus was applied using circular adhesive superficial electrodes (Ambu® Neuroline 715) in the anterior side of the carpal bones with a 2 cm distance between their centres. Supramaximal stimulation was defined as the intensity that causes visually perceptible movement of the stimulated limb, for the median nerve the thumb movement was considered. Square wave electrical pulses of 0.2 ms duration were applied at a frequency of 5.7 Hz and between 30 and 150 repetitions depending on the noise-signal ratio. SEPs were recorded in a referential fashion from the C3' (right median nerve stimulation) and C4' (left median nerve stimulation) positions and from a reference electrode at Cz' electrode (2 cm behind Cz), according to the international 10-20 system. Needle subdermal electrodes (Ambu® Neuroline subdermal) were used if patients were awake or "corkscrew" electrodes (Ambu® Neuroline corkscrew) if patients were under general anaesthesia during the procedure. The signal was digitally filtered (5-200 Hz bandpass) and analyzed within 80 msec time window poststimulus. The 30 to 150 trials were averaged to compensate for the noise. The target signal was the presence of $N_{20}$ response ipsilateral to the stroke site. Secondarily, the amplitude ($\mu$V) and latency (ms) of the $N_{20}$ response of both ipsilateral and contralateral to the stroke site were also measured. SEPs were analysed at the moment by the examiners and later on (after more than 3 months form the stroke event) by another researcher blind to the clinical outcome and the SEP monitoring result.

SEP registering started in the urgency area or in the angiography room before the femoral punction was performed and continuously during the endovascular treatment procedure. To monitor neurological damage during reperfusion, SEP monitoring was continued until the patient was transferred to the ICU or the Acute Stroke Unit.

Statistical considerations:

The adjusted predictive value of the N20 on functional Independence (mRS≤2) after MT was analysed by binary logistic regression and its predictive value on the full range of disability by ordinal logistic regression.

Prognostic variables: the main prognostic variable is the functional independency at 7-days from the stroke or at hospital discharge, defined by mRS score $\leq$ 2; which has showed great correlation with functional independency at 90-days (Davalos A et al. Lancet Neurol 2017). Other secondary prognostic variables were: functional independency at 90-days valued by the mRS scale; disability seriousness at 7 and 90 days according to the mRS scale; (dramatic) neurologic improvement, defined as a (10) 4 point decrease in the NIHSS scale compared to the baseline; optimal revascularisation defined by a TICI scale score of 2b or 3, size of the stroke in the control CT or MR; and several baseline image variables such as core, size of the ischemic volume and state of the collateral circulation.

## 2. Results

A total of 228 patients were included. Five were excluded, three because of the incapability to recover the SEP registries for the analysis performed by the blind researcher, one because a previous stroke was found in the neuroimaging study which could affect the SEPs result and a last one due to the presence of bi-hemispherical acute ischemic strokes. Therefore, final sample size was 223 patients. Average age was 69.9 years (SD 13.7) and 41.7% were women. All patients scored between 0 and 2 in the modified Rankin Scale (mRS) at the moment of its inclusion, except four of them. These four patients scored 3 in the mRS and were considered for endovascular treatment by the vascular neurologist's criteria. They had a non-vascular related previous comorbidity. Forty-eight percent of the patients presented with a stroke due to occlusion of the M1 segment of the middle cerebral artery (MCA), making this the most frequent stroke. Other types of occlusion were the internal carotid artery (ICA) with 19.3%, the tandem MCA-ICA with 19.7% and the M2 segment of the MCA. Regarding to the type of treatment received, 113 patients (51,1 %) underwent endovascular treatment after intravenous thrombolysis and 108 (48.9%) primary endovascular treatment. Median NIHSS score at arrival to the hospital was of 18 points ([12-22] 95% C.I.) and mean and median ASPECTS score of the neuroimage previous to the MT was 8. Mean time from the beginning of the symptoms to the arterial recanalization was of 432 minutes with a median of 340.5 ([240-522] 95% C.I.). The recanalization time couldn't be achieved in 26 patients, 23 due to a lack of recanalization and 3 for an unknown chronology of the symptoms.

### 2.1 Neurophysiological variables

Target neurophysiological variable is the $N_{20}$ response of the SEPs, which can be defined in a qualitative (presence/absence/not valuable) and quantitative (amplitude and latency). In the ipsilateral side of the stroke 110 patients had presence of $N_{20}$, it was absent in 58 of them and it couldn't be valued in the rest of them (55). In the contralateral side, 162 had presence of $N_{20}$, it was absent in none and it couldn't be valued in 61. We note that the $N_{20}$ response was not assessable in 25% and 27% of patients in the affected and healthy cerebral hemisphere, respectively, by ambient radiofrequency artefacts. The mean and median $N_{20}$ amplitude was lower in the affected cerebral hemisphere while the latency was similar between both hemispheres (Table 1). The mean time from symptom onset to baseline $N_{20}$ recording was 299.03 minutes (SD 267.6), while the median time was of 252 minutes, with an interquartile range (IQR) of [149.7-363.7]).

*Table 1: Baseline neurophysiological variables (intention-to-test population). $\mu$V: microvolts; ms: milliseconds.*

| Baseline neurofisiological study | | Affected brain hemisphere (ipsilateral) | Healthy brain hemisphere (contralateral) |
|---|---|---|---|
| N20 n (%) | | | |
| | Present | 110 (49.3) | 162 (72.6) |
| | Absent | 58 (26) | 0 |
| | Not valuable | 55 (24.7) | 61 (27.4) |
| N20 amplitude, $\mu$V | | | |
| | Mean (SD) | 1.7 (2.5) | 2.4 (7.4) |
| | Median (IQR) | 0.85 (0-2.3) | 1.3 (0.7-2.4) |
| N20 latency, ms | | | |
| | Mean (SD) | 21.1 (2.5) | 20.05 (3.5) |
| | Median (IQR) | 20.9 (19.2-22.7) | 19.9 (18.6-21.6) |

Neurophysiological result variables in the population under test

Seventy-seven patients (34.6%) had a good functional prognosis, defined as an mRS score $\leq 2$ at 7 days post-stroke. 133 patients (59.6%) had neurological improvement (improvement $\geq 4$ points on the NIHSS scale) and 79 (35.4%) had dramatic neurological improvement (improvement $\geq 10$ points on the NIHSS scale or NIHSS score 0-1) 24 hours after stroke.

Neurophysiological result variables in the population with valuable $N_{20}$

As the $N_{20}$ response wasn't baseline tested (prior to the MT) in 55 of the patients, the population was reduced to 168. Statistically significant differences between patients with present and absent baseline $N_{20}$ for all primary and secondary clinical outcome variables in the study were found (see Table 2).The p-value is used as the statistical significance indicator.

*Table 2: Primary and secondary clinical outcomes in the population with measurable N20 response. The mRS variable at 90 days after stroke was titratable in 101 and 56 of the patients with N20 present and absent, respectively.*

| Primary outcome variable | N20 present (n=110) | N20 absent (n=58) | P |
|---|---|---|---|
| mRS $\leq$ 2 at day 7, n (%) | 56 (50.9%) | 4 (6.9%) | <0.001 |
| **Secondary outcome variable** | | | |
| mRS $\leq$ 2 at day 90, n (%) | n= 101 73 (72.3) | n= 56 8 (14.3) | <0.001 |

**2.2 Prognostic capability at 7 days of the N20 response before the MT**

2.2.1 Good functional prognostic (mRS $\leq$ 2) at 7 days

**Post-hoc analysis**

Good functional prognostic was observed in 77 patients (34,6%). They were younger, had inferior glucose and diastolic arterial tension levels, a lower initial score in the NIHSS scale and a smaller ischemic tissue extension. (Table 3).

*Table 3: Baseline clinical and time flow variables in patients with good (mRS$\leq$2) and poor (mRS>2) functional prognosis at 7 days. Optimal functional prognosis was defined as a score $\leq$2 on the mRS scale. tPA: intravenous thrombolysis; MT: mechanical thrombectomy; LMCA: left middle cerebral artery; ICA: internal carotid artery. &U Mann-Whitney;* Chi-square.*

| Baseline clinical and time flow variables (N=223) | mRS $\leq$2 (n=77, 34.6%) | mRS>2 (n=146, 65.4%) | p |
|---|---|---|---|
| Women, n (%) | 35 (45.5) | 58 (39.7) | 0.4 |
| Age (years)<br>Mean (SD) | 67.2 (14.9) | 71.3 (12,9) | **0.03** |
| Laterality left stroke, n (%) | 41 (53,2) | 87 (59,6) | 0.36 |
| Glycaemia pre-MT (mg/dl) (n=215)<br>Mean (SD) | 114.9 (25.1) | 130.1 (50) | **0.01** |
| Systolic blood pressure (mmHg)<br>Mean (SD) | 149.7 (28.4) | 153.4 (27.3) | 0.34 |
| Diastolic blood pressure (mmHg)<br>Mean (SD) | 77.4 (14.9) | 81.9 (16.8) | **0.049** |
| NIHSS<br>Mean (SD)<br>Median ( IQR ) | 14.7(5.8)<br>14 (10-20.5) | 18.1 (4.6)<br>19 (15-22) | **<0.001**<br>**0.001** |
| tPA pre-TM (n=221) n (%) | 42 (54,5) | 72 (49,3) | 0.42 |

(continued)

| Baseline clinical and time flow variables (N=223) | mRS ≤2 (n=77, 34.6%) | mRS>2 (n=146, 65.4%) | p |
|---|---|---|---|
| ASPECTS (n=199) | | | |
| Mean (SD) | 8.6 (1.1) | 7.7 (1.8) | **0.001[&]** |
| Median ( IQR ) | 9 (8-9) | 8 (7-9) | **0.018** |
| Type of arterial occlusion, n(%) | | | |
| ACI | 13 (16.9) | 30 (20.5) | |
| Tandem ACI-ACM | 15 (19,8) | 29 (19.5) | 0.798* |
| M1 | 36 (46.8) | 71 (48.6) | |
| M2 | 13 (16.9) | 16 (11) | |
| Femoral onset-puncture time , min (n=220) | | | |
| Mean (SD) | 333.2 (209.4) | 407.6 (297.8) | 0.053 |

When relating the baseline $N_{20}$ response quantitatively, characterised by latency (ms) and amplitude (mV) values, with a good or poor functional prognosis 7 days after stroke, it was found that patients with a good functional prognosis had significantly higher amplitude (2.7 vs 1.1, p<0.001) values than patients with a poor functional prognosis in the affected cerebral hemisphere. However, no statistical difference was found for latency (20.9 vs 21.4, p = 0.32; in this analysis raw values of the latency were used, with the zero latency effect corrected). The ratio of amplitude and latency between the pathological and healthy hemispheres was not statistically significant compared to the amplitude and latency in absolute value (Table 4). Therefore, relative values (% of the healthy hemisphere) are not needed.

*Table 4: Post-hoc analysis: baseline N20 amplitude and latency according to optimal functional prognosis or not 7 days after stroke. [&]Mann-Whitney U; [¶]Non-parametric test for median comparison; [#]t-Student. In the case of the variable "N20 latency" in the pathological cerebral hemisphere, the value "0" has not been included when N20 was absent. "Discr." Latency: the variable "discriminant latency" has also been created to obviate the effect of the "0" values when the N20 response is absent. It consists of the difference in absolute value with respect to the normal latency of the N20 response (20±5 ms). In the variable " N20 amplitude pathological cerebral hemisphere", the value "0" has been included when the N20 response was absent. [x]In the variable "N20 latency pathological cerebral hemisphere", the "0" latency values assigned to patients whose N20 response was absent were omitted. Therefore, this variable defines the average latency of the N20 response for patients whose N20 response was present, with the other patients excluded.*

| Baseline N20 | mRS ≤ 2 n= 60 | mRS >2 n= 108 | P |
|---|---|---|---|
| N20 amplitude pathological cerebral hemisphere (μV) | | | |
| Mean (SD) | 2,9 (3,3) | 2,3 (1,9) | **<0,001[&]** |
| Median (Quartiles) | 1,6 (0,9-3,2) | 0 (0-1,8) | **<0,00[1]** |
| N20 latency pathological cerebral hemisphere[x] (ms) | | | |
| Mean (SD) | 20,9 (2,7) | 21,4 (2,3) | 0,322[#] |
| Median (Quartiles) | 20,4 (19-22,6) | 21,2 (19,7-23) | |
| N20 "discr" latency pathological cerebral hemisphere (ms) Mean (SD) | 3,4 (5) / 1,8 (0,9-3,1) | 11,2 (9) / 20 (1,7-20) | **<0,001[&]** / **<0,001[¶]** |
| N20 amplitude healthy cerebral hemisphere (μV) | | | |
| Mean (SD) | 1,9 (1,7) | 2,6 (9,3) | 0,641[#] |
| Median (Quartiles) | 1,4 (0,8-2,3) | 1,3 (0,7-2,3) | |
| N20 latency healthy cerebral hemisphere (ms) | | | |

(continued)

| Baseline N20 | mRS $\leq$ 2 n= 60 | mRS >2 n= 108 | P |
|---|---|---|---|
| Mean (SD) | 19,7 (3,9) | 20,3 (2,9) | 0,170[#] |
| Median (Quartiles) | 19,7(18,4-21,5) | 20(18,8-22,2) | |
| Ratio amplitude N20 pathological side/ healthy side-100 Mean (SD) Median (Quartiles) | 164,5 (170,4) 111,8 (60-226,9) | 93,1 (157,6) 0 (0-139,6) | **<0,001[&]** **<0,001[¶]** |

When relating 7-day functional prognosis to baseline $N_{20}$ response measured categorically (presence/absence), of the 58 patients with absent baseline $N_{20}$ response on the ipsilateral side of the stroke, 54 had a poor clinical prognosis (mRS>2). On the other hand, of the 110 patients with baseline $N_{20}$ response present on the ipsilateral side of the stroke, 56 had a good clinical prognosis. $N_{20}$ was non-assessable in 22.1% and 26% of patients with good and poor clinical prognosis, respectively (Table 5). Taking into account these results and excluding patients with non-assessable $N_{20}$, the presence of baseline $N_{20}$ has a sensitivity for predicting an optimal functional prognosis 7 days after stroke of 93% and a specificity of 50% with a positive and negative predictive value of 51% and 93%, respectively (Table 6). Therefore, the measurement of the $N_{20}$ response on the ipsilateral side of the stroke can be a reliable way of determining that a patient won't benefit from having an endovascular treatment such as a mechanical thrombectomy performed.

*Table 5: Primary outcome variable: predictive capacity of baseline N20 response for functional prognosis 7 days after stroke.*

| N20 basal | mRS $\leq$ 2 (n=77, 34.6%) | mRS >2 (n=146, 65.4%) | Total (n=223) |
|---|---|---|---|
| Present, n (%) | 56 (77.7) | 54 (37.0) | 110 (49.3) |
| Absent, n (%) | 4 (5.2) | 54 (37.0) | 58 (26.0) |
| Not valuable, n (%) | 17 (22.1) | 38 (26.0) | 55 (24.7) |

*Table 6: Accuracy of the N20 response for predicting optimal functional prognosis 7 days after stroke. CI, confidence interval; PPV, positive predictive value; NPV, negative predictive value.*

| Sensitivity 95%CI | Specificity 95%CI | NPV 95%CI | PPV 95%CI |
|---|---|---|---|
| 0.93 (0.78-0.98) | 0.5 (0.24-0.59) | 0.51 (0.41-0.56) | 0.93 (0.80-0.98) |

**Univariate analysis**

When relating the clinical variables considered in the study to an optimal functional prognosis 7 days after stroke, it was observed that the variables related to $N_{20}$, have a better ability to predict an optimal functional prognosis than other variables used in routine clinical practice such as the NIHSS or ASPECTS scales (Table 7). It is noted the predictive power of the dichotomised $N_{20}$ amplitude (odds ratio (OR) = 14, Area Under the [ROC] Curve (AUC) = 0.713) and of the log-transformed $N_{20}$ amplitude (OR=1.93, AUC = 0.726).

*Table 7: Univariate analysis. Predictive capacity of clinical variables for optimal functional prognosis at 7 days. *All the neurophysiological variables refer to the cerebral hemisphere affected by the stroke. rTPA: intravenous fibrinolysis. "Discr." Latency: the variable "discriminant latency" has also been created to obviate the effect of the "0" values when the N20 response is absent. It consists of the difference in absolute value with respect to the normal latency of the N20 response (20 $\pm$ 5 ms). In the variable "amplitude N20 pathological cerebral hemisphere", the value "0" has been included when the N20 response was absent.*

| Clinical variables | Raw OR | 95% IC | AUC | p |
|---|---|---|---|---|
| Women, n(%) | 0.978 | 0,959-0,998 | 0,590 | 0,032 |
| Age (years) | 1.264 | 0.722-2,110 | 0,529 | 0,410 |
| Glycaemia (per unit, mg/dl) | 0,990 | 0,980-0,998 | 0,553 | 0,009 |

(continued)

| Clinical variables | Raw OR | 95% IC | AUC | p |
|---|---|---|---|---|
| Mean arterial pressure (per unit, mmHg) | 0,985 | 0.969-1.002 | 0,573 | 0,079 |
| NIHSS | 0,881 | 0,832-0,931 | 0,670 | <0,001 |
| ASPECTS | 1,478 | 1,188-1,886 | 0,636 | <0,001 |
| Previous treatment with rTPA | 1,188 | 0,681-2,078 | 0,521 | 0,544 |
| Presence N20 (yes)* | 14 | 5,283-48,567 | 0,713 | <0,001 |
| Amplitude N20 (per unit, $\mu$V)* | 1,307 | 1,130-1,555 | 0,726 | <0,001 |
| Amplitude logN20* | 1,925 | 1,508-2,529 | 0,726 | <0,001 |
| Latency N20 (per unit, ms)* | 0,873 | 0,821-0,918 | 0,724 | <0,001 |

**Multivariate analysis**

A multivariate analysis was performed including all significant variables with p<0.1. In multivariate model A the log-transformed amplitude of $N_{20}$ was included while in the multivariate model B the dichotomic (present/absent) amplitude of $N_{20}$ was used. Multivariate analysis showed that both log-transformed (model A) and dichotomic (model B) $N_{20}$ amplitude response behaved as an independent factors associated with good functional prognosis at 7 days (OR 1.83; [1,35-2,57]; p<0.001 in model A and OR 9.88 [3,08-44.58]; p=0.001 in model B). (Table 8).

*Table 8: Multivariate analysis: clinical variables and optimal functional prognosis 7 days after stroke. *All neurophysiological variables refer to the cerebral hemisphere affected by the stroke.*

| | Model A | | Model B | |
|---|---|---|---|---|
| | OR (IC 95%) | p | OR (IC 95%) | p |
| Age | 0,97 (0,94-1.00) | 0.087 | 0,97 (0,95-1.00) | **0.027** |
| NIHSS | 0,88 (0,81-0,95) | **0.002** | 0,90 (0,84-0,96) | **0.002** |
| Glycaemia (per unit, mg/dl) | 0,99 (0.98-1.01) | 0.366 | 0,99 (0.98-1.01) | 0,235 |
| Mean arterial pressure (mmHg) | 0,98 (0.96-1.01) | 0.186 | 0,98 (0.96-1.01) | 0.148 |
| ASPECTS | 1,37 (1,01-1,92) | **0.055** | 1,34 (1,03-1,79) | **0,036** |
| Amplitude logN20* | 1,83 (1,35-2,57) | **<0.001** | | |
| Presence N20 (yes)* | | | 9,88 (3,08-44,58) | 0,001 |

Logistic regression models have also been constructed for different clinical variables of interest in isolation or in combination: baseline clinical variables (age, sex, laterality, blood glucose and mean arterial pressure (MAP), NIHSS, ASPECTS and N20 response (see Table 9). The N20 response has been included in several configurations: the amplitude of the SEP using the variable in a quantitative way (quantitative amplitude), the same amplitude but applying a logarithmic transformation to the values (quantitative log-transformed amplitude), and the amplitude but using the variable in a dichotomised way, i.e. either indicating it is present or absent, according to a threshold (dichotomised amplitude). The models were designed as follows:

- Model 0a: NIHSS
- Model 0b: NIHSS + baseline variables
- Model 1a: quantitative N20 amplitude
- Model 1b: NIHSS basal + quantitative N20 amplitude + baseline variables
- Model 2a: quantitative N20 log-transformed amplitude
- Model 2b: NIHSS + quantitative N20 log-transformed amplitude + baseline variables
- Model 3a: dichotomised N20 amplitude

- Model 3b: NIHSS + dichotomised N20 amplitude + baseline variables
- Model 4: NIHSS + ASPECTS
- Model 5: NIHSS + dichotomised N20 amplitude

*Table 9: Models logistic regression for 7-day prediction before the MT: number of patients and AUC values.*

|  | N | AUC |
|---|---|---|
| Modelo 0a | 223 | 0.670 |
| Modelo 0b | 213 | 0.750 |
| Modelo 1a | 168 | 0.726 |
| Modelo 1b | 161 | 0.797 |
| Modelo 2a | 168 | 0.726 |
| Modelo 2b | 161 | 0.823 |
| Modelo 3a | 168 | 0.717 |
| Modelo 3b | 161 | 0.830 |
| Modelo 4 | 199 | 0.719 |
| Modelo 5 | 168 | 0.784 |

The quantitative $N_{20}$ amplitude response alone (Model 1a) has a higher predictive ability for good functional prognosis 7 days after stroke than the NIHSS scale (Model 0a) (AUC 0.726 vs. 0.670) and also higher than the combination of the NIHSS and ASPECTS scales (Model 4) (AUC 0.719), the clinical variables currently used for the selection of candidates for endovascular treatment. Notably, while the NIHSS scale can be assessed in a pre-hospital setting, the ASPECTS scale requires an imaging test, either CT or MRI of the brain, and is therefore restricted to a hospital setting. Furthermore, the combination of NIHSS and dichotomised $N_{20}$ amplitude, (Model 5); the combination of NIHSS, baseline and log transformed quantitative $N_{20}$ amplitude variables (Model 2b) and the combination of NIHSS, baseline and dichotomised $N_{20}$ amplitude variables have an AUC of 0.784, 0.823 and 0.830, respectively; both higher than that of the NIHSS alone and the combination of NIHSS and ASPECTS (Model 4 - AUC 0.719) (see Figure 3 A-F for the models ROC curves and Table 9 for the models regressions AUC). It can be concluded that adding N20 to NIHSS and usual baseline variables significantly increases its predictive ability.

A further analysis of the model 1a was performed to determine the current amplitude values that better determine the difference between a good and bad outcome, i.e. the values that provide the best determination thresholds. Several threshold cuts were made and the sensitivity specificity, PPV and NPV were calculated for each of them (see Table 10).

*Table 10: Classification performance pre-intervention at 7 days for different threshold cuts*

| Cut | Prob | Sensitivity Mean (95% CI) | Specificity Mean (95% CI) | PPV Mean (95% CI) | NPV Mean (95% CI) |
|---|---|---|---|---|---|
| 0 | 0.21 | 1.00 (1.00-1.00) | 0.00 (0.00-0.00) | 0.36 (1.00-0.36) | NaN (1.00-NaN) |
| 0.25 | 0.28 | 0.92 (0.73-0.98) | 0.49 (0.23-0.62) | 0.46 | 0.87 |
| 0.375 | 0.279 | 0.933 | 0.528 | 0.523 | 0.934 |
| 0.5 | 0.37 | 0.90 (0.70-0.98) | 0.53 (0.35-0.64) | 0.46 | 0.84 |
| 0.75 | 0.48 | 0.78 (0.62-0.95) | 0.61 (0.47-0.73) | 0.46 | 0.76 |
| 1 | 0.58 | 0.72 (0.47-0.87) | 0.67 (0.53-0.77) | 0.45 | 0.73 (0.70-0.80) |
| 1.25 | 0.68 | 0.60 (0.32-0.82) | 0.70 (0.59-0.80) | 0.46 | 0.71 (0.62-0.77) |
| 1.5 | 0.76 | 0.53 (0.28-0.78) | 0.74 (0.65-0.83) | 0.41 | 0.67 (0.50-0.72) |
| 1.75 | 0.83 | 0.45 (0.23-0.73) | 0.78 (0.68-0.86) | 0.37 | 0.65 (0.40-0.69) |
| 2 | 0.88 | 0.40 (0.20-0.68) | 0.78 (0.68-0.86) | 0.38 | 0.65 (0.40-0.70) |
| 2.25 | 0.92 | 0.35 (0.18-0.63) | 0.80 (0.70-0.90) | 0.36 (0.35-0.49) | 0.64 (0.35-0.69) |

(continued)

| Cut | Prob | Sensitivity Mean (95% CI) | Specificity Mean (95% CI) | PPV Mean (95% CI) | NPV Mean (95% CI) |
|---|---|---|---|---|---|
| 2.5 | 0.94 | 0.35 (0.18-0.63) | 0.83 (0.73-0.94) | 0.30 (0.28-0.44) | 0.63 (0.28-0.67) |
| 2.75 | 0.96 | 0.30 (0.17-0.53) | 0.83 (0.73-0.94) | 0.33 (0.28-0.47) | 0.63 (0.28-0.67) |
| 3 | 0.97 | 0.27 (0.13-0.47) | 0.86 (0.74-0.96) | 0.31 (0.25-0.47) | 0.63 (0.25-0.67) |
| 3.5 | 0.99 | 0.22 (0.08-0.35) | 0.88 (0.77-0.98) | 0.34 (0.22-0.52) | 0.64 (0.22-0.67) |
| 4 | 1 | 0.15 (0.07-0.30) | 0.95 (0.83-1.00) | 0.31 (0.15-0.53) | 0.64 (0.15-0.66) |
| 7 | 1 | 0.13 (0.03-0.23) | 1.00 (0.90-1.00) | 0.30 (0.08-0.62) | 0.64 (0.08-0.66) |

It was observed that a classifier with an absolute N20 amplitude threshold value of 0.375 $\mu$V of the N20 response has a sensitivity and NPV of 93% and a specificity and PPV of 52%; and a classifier with an absolute N20 amplitude threshold value between 0.25 and 0.5 $\mu$V of the N20 response has a sensitivity and NPV of between 90-92%, a specificity between 49-53% and a PPV value of 46%.

For an N20 SEP absolute amplitude threshold comprised between 0.25 and 0.5, $\mu$V an optimum sensitivity and acceptable specificity is obtained for a functional prognostic before an endovascular stroke treatment at 7 days. The most optimal sensitivity and specificity is obtained for an N20 SEP absolute amplitude threshold is comprised between 0.37-0.38 $\mu$V.

2.2.2 Functional prognostic (shift analysis) at 7 days

**Post-hoc analysis**

Patients with $N_{20}$ present have an overall better functional prognosis in all categories of the mRS scale. Fifty-one percent of patients with present $N_{20}$ response had a score of 0-2 on the mRS scale at 7 days post-stroke, while this was only 15.4% in the group of patients with baseline $N_{20}$ absent. Notably, the percentage of patients with mRS scores of 4 and 5-6 is clearly lower in patients with $N_{20}$ present (15.5 vs 20.7% and 21.9 vs 63.9%, respectively).

**Univariate analysis**

When relating the clinical variables assessed in the study to the functional prognosis according to all degrees of disability at 7 days, it was observed that the variables related to $N_{20}$, especially the dichotomised $N_{20}$ amplitude, have a better capacity to predict an optimal functional prognosis than other variables used in routine clinical practice such as the NIHSS or ASPECTS scales (Table 11).

*Table 11: Univariate analysis. Predictive capacity of 7-day functional prognosis of the clinical variables. The OR and Brier coefficient (low values indicate better predictive capacity) are shown for the clinical variables of the study. The variables are ordered according to the Brier coefficient. *All neurophysiological variables refer to the cerebral hemisphere affected by the stroke. rTPA: intravenous fibrinolysis. #N20 latency has been corrected to avoid the effect of the value "0".*

| Clinical variables | Raw OR | 95% CI | Brier | p |
|---|---|---|---|---|
| Latency N20* (ms)# | 0.894 | 0.861-0.926 | 0.190 | <0.001 |
| Amplitude logN20 | 1.771 | 1.461-2.164 | 0.192 | <0.001 |
| Presence N20 (yes)* | 8.281 | 1.937-4.401 | 0.193 | <0.001 |
| NIHSS | 0.895 | 0.855-0.936 | 0.204 | <0.001 |
| Amplitude N20 (per unit, $\mu$V)* | 1.237 | 1.102-1.405 | 0.211 | <0,001 |
| ASPECTS | 1.426 | 1.205-1.707 | 0.212 | <0,001 |
| Age | 0.979 | 0.963-0.995 | 0.222 | 0.009 |
| Glycaemia | 0.988 | 0.981-0.994 | 0.222 | <0.001 |

(continued)

| Clinical variables | Raw OR | 95% CI | Brier | p |
|---|---|---|---|---|
| Mean arterial pressure (MAP) | 0.990 | 0.977-1.003 | 0.223 | 0.140 |
| rTPA+TM | 1.326 | 0.827-2.131 | 0.225 | 0.241 |
| Left laterality (yes) | 0.946 | 0.584-1.531 | 0.226 | 0.820 |
| Sex (woman) | 1.064 | 0.662-1.711 | 0.226 | 0.798 |
| Occlusion ACM-M1 | 1.288 | 0.682-0.585 | 0.226 | 0.563 |
| Occlusion ACM-M2 | 1.388 | 0.682-0.585 | 0.226 | 0.563 |
| Occlusion-tandem | 0.872 | 0.682-0.585 | 0.226 | 0.563 |

**Multivariate analysis**

A similar multivariate analysis was performed as in the good functional prognostic analysis, including all significant variables with p<0.1 and model A (including the log-transformed amplitude of $N_{20}$ ($\mu$V)) and model B (including the categoric (present/absent) amplitude of $N_{20}$ (%)) were designed. The multivariate analysis showed that the baseline variables associated with the ordinal mRS at 7 days are age (older age, worse outcome), baseline NIHSS (higher baseline severity, worse outcome), blood glucose (higher values, worse outcome), ASPECTS (higher values associated with better outcome) and baseline amplitude or presence of the $N_{20}$ response (values present are associated with better outcome than absent values) (Table 12).

*Table 12: Multivariate analysis: clinical variables and functional prognosis 7 days after stroke. \*All neurophysiological variables refer to the cerebral hemisphere affected by the stroke.*

| | Model A | | Model B | |
|---|---|---|---|---|
| | OR (IC 95%) | p | OR (IC 95%) | p |
| Age | 0.97 (0.94-0.99) | 0.015 | 0.98 (0.96-0.99) | 0.009 |
| NIHSS | 0.92 (0.87-0.98) | 0.009 | 0.93 (0.88-0.98) | 0.007 |
| Glycaemia (per unit, mg/dl) | 0.99 (0.98-1.00) | 0.048 | 0.99 (0.98-1.00) | 0,004 |
| ASPECTS | 1.40 (1.12-1.77) | 0.004 | 1.31 (1.08-1.60) | 0.007 |
| Amplitude logN20* | 1.65 (1.32-2.09) | <0.001 | | |
| Presence N20 (yes)* | | | 6.41 (2.98-14.29) | 0.001 |

**2.3 Prognostic capability at 90 days of the N20 response before the MT**

2.3.1 Good functional prognostic (mRS $\leq$ 2) at 90 days

**Post-hoc analysis**

The mRS was evaluated at 90 days for 204 patients .Good functional prognostic was observed in 98 patients (48%). They were younger, had inferior glucose and diastolic arterial tension levels, a lower initial score in the NIHSS scale and a smaller ischemic tissue extension. (Table 13).

*Table 13: Baseline clinical and time flow variables in patients with good (mRS≤2) and poor (mRS>2) functional prognosis at 90 days. Optimal functional prognosis is defined as a score ≤2 on the mRS scale. tPA: intravenous thrombolysis; MT: mechanical thrombectomy; LMCA: left middle cerebral artery; ICA: internal carotid artery. &U Mann-Whitney;\* Chi-square.*

| Baseline clinical and time flow variables (N=204) | mRS ≤2 (n=98, 48%) | mRS>2 (n=106, 52%) | p |
|---|---|---|---|
| Women, n(%) | 41 (41.8) | 44(41.5) | 0.962 |
| Age (years) <br> Mean (SD) | 67.6 (13.1) | 74.5 (11.6) | |
| Laterality left stroke, n(%) | 53 (54.1) | 60 (56.6) | 0.412 |
| Glycaemia pre-MT (mg/dl) (n=215) <br> Mean (SD) | 115 (25.1) | 136.2 (50) | **0.001** |
| Systolic blood pressure (mmHg) <br> Mean (SD) | 152.04 (26.1) | 153.3 (28.4) | 0.750 |
| Diastolic blood pressure (mmHg) <br> Mean (SD) | 78.9 (14.9) | 82 (16.7) | 0.161 |
| NIHSS <br> Mean (SD) <br> Median ( IQR ) | 15.1(5.6) <br> 14 (10-20.5) | 18.6 (4.4) <br> 19 (15-22) | **<0.001** <br> **<0.001&** |
| tPA pre-TM (n=221) n (%) | 55 (56.1) | 44 (41.5) | 0.093 |
| ASPECTS (n=199) <br> Mean (SD) <br> Median ( IQR ) | 8.3 (1.3) <br> 9 (8-9) | 7.6 (1.8) <br> 8 (7-9) | **0.004&** <br> **0.009** |
| Type of arterial occlusion, n(%) <br> ACI <br> Tandem ACI-ACM <br> M1 <br> M2 | 15 (15.3) <br> 23 (23.4) <br> 45 (45.9) <br> 15 (15.3) | 25 (23.6) <br> 15(14.1) <br> 54 (50.9) <br> 12(11.3) | 0.130* |
| Femoral onset-puncture time, min (n=220) <br> Mean (SD) | 336.3 (221.2) | 418 (303.4) | **0.032** |

As in the 7-day prognosis analysis, it was found that patients with a good functional prognosis 90 days after stroke had significantly higher amplitude (2.2 vs 1.1, p=0.004) values than patients with a poor functional prognosis 90 days after stroke in the affected cerebral hemisphere. However, no statistical difference was found for latency (21.1 vs 21.3, p = 0.72). The ratio of amplitude and latency between the pathological and healthy hemispheres was not statistically significant compared to the amplitude and latency in absolute value (Table 14). Equivalently, relative values (% of the healthy hemisphere) are not needed.

*Table 14: Post-hoc analysis: baseline N20 amplitude and latency according to optimal functional prognosis or not 90 days after stroke. &Mann-Whitney U; ¶Non-parametric test for median comparison; #t-Student. In the case of the variable "N20 latency" in the pathological cerebral hemisphere, the value "0" has not been included when N20 was absent. "Discr." Latency: the variable "discriminant latency" has also been created to obviate the effect of the "0" values when the N20 response is absent. It consists of the difference in absolute value with respect to the normal latency of the N20 response (20±5 ms). In the variable "amplitude N20 pathological cerebral hemisphere", the value "0" has been included when the N20 response was absent.*

| Baseline N20 | mRS ≤ 2 n=81 | mRS >2 n=76 | P |
|---|---|---|---|
| N20 amplitude pathological cerebral hemisphere (μV) <br> Mean (SD) | 2,4 (3,1) | 1,1 (2) | **<0,001&** |

(continued)

| Baseline N20 | mRS ≤ 2 n=81 | mRS >2 n=76 | P |
|---|---|---|---|
| Median (Quartiles) | 1,3 (0,7-3) | 0 (0-1,1) | **<0,001¶** |
| N20 latency pathological cerebral hemisphere (ms) Mean (SD) Median (Quartiles) | 21,1 (2,6) 20,4 (19,2-22,7) | 21,3 (2,4) 21,2 (19,8-22,7) | 0,724# |
| N20 "discr" latency pathological cerebral hemisphere (ms) Mean (SD) | 4,1 (5,9) 2 (0,8-3,7) | 13,6 (8,7) 20 (2,7-20) | **<0,001&** **<0,001¶** |
| N20 amplitude healthy cerebral hemisphere (µV) Mean (SD) Median (Quartiles) | 1,7 (1,5) 1,3 (0,8-1,9) | 3,2 (11,1) 1,4 (0,8-2,7) | 0,321# |
| N20 latency healthy cerebral hemisphere (ms) Mean (SD) Median (Quartiles) | 20,3 (2,8) 19,9 (18,6-21,5) | 20,4 (2,9) 19,9 (18,9-22,4) | 0,202# |
| Ratio amplitude N20 pathological side/ healthy side-100 Mean (SD) Median (Quartiles) | 165,5 (181,6) 108,6 (50,2-228,2) | 72 (143,1) 0 (0-109,4) | **<0,001&** **<0,001¶** |

When relating 90-day functional prognosis to baseline $N_{20}$ response measured categorically (presence/absence), of the 56 patients with absent baseline $N_{20}$ response on the ipsilateral side of the stroke, 48 had a poor clinical prognosis (mRS>2). On the other hand, of the 101 patients with baseline $N_{20}$ response present on the ipsilateral side of the stroke, 73 had a good clinical prognosis. $N_{20}$ was non-assessable in 17.3% and 28,3% of patients with good and poor clinical prognosis, respectively (Table 15). Taking into account these results and excluding patients with non-assessable N20, the presence of baseline N20 has a sensitivity for predicting an optimal functional prognosis 7 days after stroke of 90.1% and a specificity of 63.2% with a positive and negative predictive value of 85,7% and 72.3%, respectively (Table 16). Therefore, the measurement of the N20 response on the ipsilateral side of the stroke can be a reliable way of prognosticating the outcome of a patient having an endovascular treatment such as a mechanical thrombectomy performed.

Table 15: *Predictive ability of baseline categorical N20 response) of functional prognosis 90 days after stroke.*

| Baseline N20 | mRS ≤ 2 (n=98, 34.5%) | mRS >2 (n=106, 65.4%) | Total (n=204) |
|---|---|---|---|
| Present, n (%) | 73 (74.5) | 28 (26.4) | 101 (49.5) |
| Absent, n (%) | 8 (8.2) | 48 (45.3) | 56 (27.5) |
| Not valuable, n (%) | 17 (17.3) | 30 (28.3) | 47(23) |

Table 16: *Accuracy of the N20 response for predicting optimal functional prognosis 7 days after stroke. CI, confidence interval; PPV, positive predictive value; NPV, negative predictive value.*

| Sensitivity 95%IC | Specificity 95%IC | NPV 95%IC | PPV 95%IC |
|---|---|---|---|
| 0.901 (-) | 0.632 (-) | 0.723 (-) | 0.857 (-) |

**Univariate analysis**

When relating the clinical variables considered in the study to an optimal functional prognosis 90 days after stroke, it was equally observed that the variables related to $N_{20}$, have a better ability to predict an optimal functional prognosis than other variables used in routine clinical practice such as the NIHSS or ASPECTS scales (Table 17). It is noted the predictive power of the dichotomised $N_{20}$ amplitude (OR = 15.62) and the log-transformed $N_{20}$ amplitude (OR=1.95).

*Table 17: Predictive capacity of clinical variables for optimal functional prognosis at 90 days. *All neurophysiological variables refer to the cerebral hemisphere affected by the stroke. #t-Student. In the case of the variable "N20 latency" in the pathological cerebral hemisphere, the value "0" has not been included when N20 was absent. rTPA: intravenous fibrinolysis.*

| Clinical variables | Raw OR | 95% IC | AUC | p |
|---|---|---|---|---|
| Women, n(%) | 1,01 | 0,58-1,76 | 0,502 | 0,962 |
| Age (years) | 0,95 | 0,93-0,97 | 0,670 | **<0,001** |
| Glycaemia (per unit, mg/dl) | 0,99 | 0,97-0,99 | 0,600 | **<0,001** |
| Mean arterial pressure (per unit, mmHg) | 0.99 | 0.97-1.00 | 0.528 | 0.288 |
| NIHSS | 0.87 | 0.82-0.92 | 0.678 | **<0.001** |
| ASPECTS | 1.33 | 1.09-1.63 | 0.608 | **0.003** |
| Previous treatment with rTPA | 1.81 | 1.04-3.18 | 0.574 | **0.035** |
| Presence N20 (yes)* | 15.62 | 6.90-40 | 0.768 | **<0.001** |
| Amplitude N20 (per unit, $\mu$V)#* | 1.25 | 1.07-1.51 | 0.737 | **0.003** |
| Amplitude logN20# | 1,25 | 1,07-1,51 | 0,737 | **0,003** |
| Latency N20 "discr"* (per unit, ms)* | 0.86 | 0.82-0.90 | 0.770 | **<0.001** |

**Multivariate analysis**

A multivariate analysis was performed. In multivariate model A all significant variables with p<0.1 were included, including the amplitude of $N_{20}$ was used while in the multivariate model B an automatic variable selection process (AIC) was used including the dichotomic (present/absent) amplitude of $N_{20}$. Multivariate analysis showed that in both models dichotomic $N_{20}$ amplitude response behaved as an independent factor associated with good functional prognosis at 90 days (OR 15.36; [5,50-49.87]; p<0.001 in models A and B). (Table 18).

*Table 18: Multivariate analysis: clinical variables and functional prognosis 90 days after stroke. *All neurophysiological variables refer to the cerebral hemisphere affected by the stroke.*

| | Model A | | Model B | |
|---|---|---|---|---|
| | OR (IC 95%) | p | OR (IC 95%) | p |
| Age | 0.94 (0.91-0.97) | **<0.001** | 0.93 (0.91-0.96) | **<0.001** |
| NIHSS | 0.91 (0.83-0.99) | **0.037** | 0.91 (0.84-0.98) | **0.018** |
| Previous treatment with rTPA | 1,92 (0,81-4,63) | 0,140 | 1,58 (0,73-3,45) | 0,249 |
| Glycaemia (per unit, mg/dl) | 0,99 (0,97-1) | 0,128 | 0,99 (0,98-1) | 0,082 |
| ASPECTS | 1,33 (0,98-1,84) | 0,077 | 1,17 (0,89-1,57) | 0,268 |
| Amplitude logN20* | 1,79 (1,36-2,43) | **<0,001** | | |
| Presence N20 (yes)* | | | 15,36 (5,50-49,87) | **<0,001** |

Logistic regression models have also been constructed for different clinical variables of interest in isolation or in combination: baseline clinical variables (age, sex, laterality, blood glucose and mean arterial pressure (MAP), NIHSS, ASPECTS and N20 response (see Table 19). The N20 response has been included in several configurations: the amplitude of the SEP using the variable in a quantitative way (quantitative amplitude), the same amplitude but applying a logarithmic transformation to the values (quantitative log-transformed amplitude), and the amplitude but using the variable in a dichotomised way, i.e. either indicating it is present or absent, according to a threshold (dichotomised amplitude). The models were designed as follows:

- Model 0a: NIHSS
- Model 0b: NIHSS + baseline variables
- Model 1a: quantitative N20 amplitude
- Model 1b: NIHSS basal + quantitative N20 amplitude + baseline variables
- Model 2a: quantitative N20 log-transformed amplitude
- Model 2b: basal NIHSS + quantitative N20 log-transformed amplitude + baseline variables
- Model 3a: dichotomised N20 amplitude
- Model 3b: baseline NIHSS + dichotomised N20 amplitude + baseline variables
- Model 4: NIHSS basal + ASPECTS

*Table 19: Models logistic regression for 90 day prediction before the MT: number of patients and AUC values.*

|  | N | AUC |
|---|---|---|
| Modelo 0a | 204 | 0.678 |
| Modelo 0b | 197 | 0.793 |
| Modelo 1a | 157 | 0.737 |
| Modelo 1b | 152 | 0.817 |
| Modelo 2a | 157 | 0.737 |
| Modelo 2b | 152 | 0.851 |
| Modelo 3a | 157 | 0.766 |
| Modelo 3b | 152 | 0.895 |
| Modelo 4 | 184 | 0.697 |

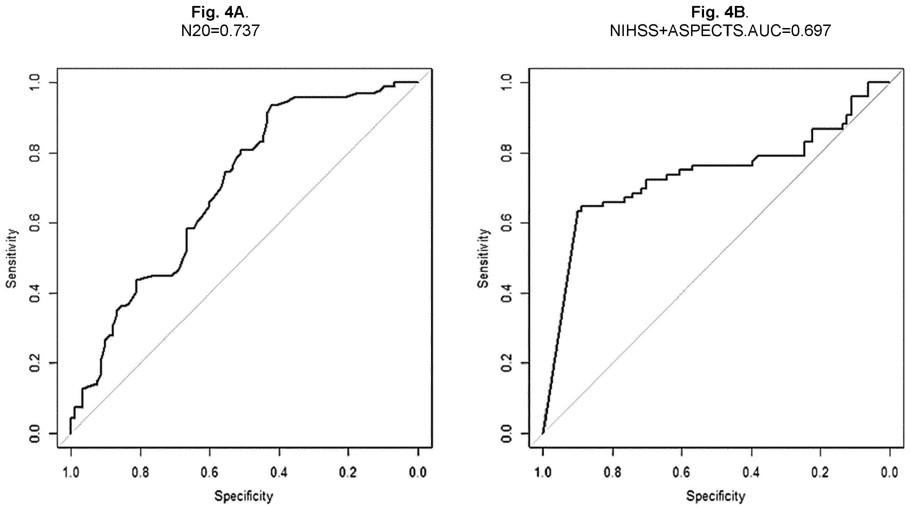

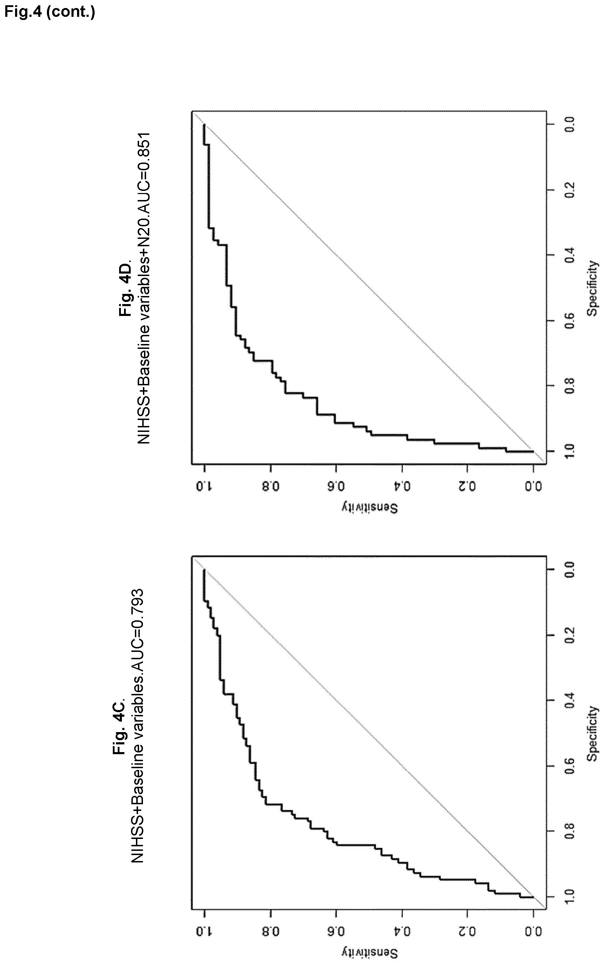

The quantitative $N_{20}$ responsealone (Model 1a) still has a higher predictive ability for good functional prognosis 90 days after stroke than the NIHSS scale (Model 0a) (AUC 0.736 vs. AUC 0.678) and the combination of the NIHSS and ASPECTS scales (Model 4)(AUC 0.697), the clinical variables currently used for the selection of candidates for endovascular treatment. Furthermore, the combination of the NIHSS, baseline and $N_{20}$ variables has a better predictive ability (AUC 0.851 - Model 2b) (AUC 0.895 - Model 3b) than that of the combination of NIHSS and ASPECTS (Model 4)(AUC 0.697) and the combination of the NIHSS and basal variables (AUC 0.793) (see Figure 4 A-D for the models ROC curves and Table 19 for the models regressions AUC).

As in the 7-day outcome analysis, adding N20 to NIHSS and usual baseline variables significantly increases their predictive ability, especially when N20 is measured qualitatively or dichotomously (presence/absence) (Model 3b).

A further analysis of the model 1a was performed to determine the current amplitude values that better determine the difference between a good and bad outcome, i.e. the values that provide the best determination thresholds. Several threshold cuts were made and the sensitivity specificity, PPV and NPV were calculated for each of them (see Table 20).

**Table 20**: Classification performance pre-intervention at 90 days for different threshold cuts

| Cut | Prob | **Sensitivity** Mean (95% CI) | **Specificity** Mean (95% CI) | **PPV** Mean (95% CI) | **NPV** Mean (95% CI) |
|---|---|---|---|---|---|
| 0 | 0.21 | 1.00 (1.00-1.00) | 0.00 (0.00-0.00) | 0.52 | NaN (1.00-NaN) |
| 0.25 | 0.28 | 0.88 (0.60-0.95) | 0.62 (0.41-0.75) | 0.66 | 0.76 |
| 0.339 | 0.551 | 0.889 | 0.645 | 0.65 | 0.727 |

(continued)

| Cut | Prob | Sensitivity Mean (95% CI) | Specificity Mean (95% CI) | PPV Mean (95% CI) | NPV Mean (95% CI) |
|---|---|---|---|---|---|
| 0.5 | 0.37 | 0.86 (0.57-0.95) | 0.64 (0.51-0.76) | 0.65 | 0.72 |
| 0.75 | 0.48 | 0.73 (0.27-0.94) | 0.70 (0.58-0.82) | 0.64 | 0.62 |
| 1 | 0.58 | 0.64 (0.20-0.90) | 0.74 (0.63-0.84) | 0.63 | 0.57 |
| 1.25 | 0.68 | 0.53 (0.19-0.84) | 0.76 (0.66-0.86) | 0.60 (0.51-0.69) | 0.53 (0.51-0.59) |
| 1.5 | 0.76 | 0.53 (0.19-0.84) | 0.78 (0.67-0.87) | 0.55 (0.42-0.65) | 0.50 (0.42-0.55) |
| 1.75 | 0.83 | 0.35 (0.14-0.78) | 0.79 (0.70-0.88) | 0.50 (0.33-0.63) | 0.48 (0.33-0.53) |
| 2 | 0.88 | 0.35 (0.14-0.78) | 0.79 (0.70-0.89) | 0.48 (0.31-0.61) | 0.47 (0.31-0.52) |
| 2.25 | 0.92 | 0.35 (0.14-0.78) | 0.80 (0.71-0.92) | 0.44 (0.27-0.58) | 0.46 (0.27-0.50) |
| 2.5 | 0.94 | 0.35 (0.14-0.78) | 0.83 (0.71-0.93) | 0.41 (0.25-0.56) | 0.45 (0.25-0.50) |
| 2.75 | 0.96 | 0.26 (0.11-0.72) | 0.83 (0.71-0.93) | 0.44 (0.25-0.59) | 0.46 (0.25-0.50) |
| 3 | 0.97 | 0.25 (0.10-0.67) | 0.84 (0.72-0.93) | 0.42 (0.22-0.58) | 0.46 (0.22-0.50) |
| 3.5 | 0.99 | 0.19 (0.07-0.44) | 0.87 (0.76-0.96) | 0.42 (0.19-0.60) | 0.47 (0.19-0.50) |
| 4 | 1 | 0.14 (0.05-0.28) | 0.92 (0.80-1.00) | 0.37 (0.12-0.59) | 0.47 (0.12-0.49) |
| 7 | 1 | 0.09 (0.02-0.19) | 1.00 (0.89-1.00) | 0.30 (0.06-0.62) | 0.47 (0.06-0.49) |

It was observed that a classifier with an absolute N20 amplitude threshold value between 0.339 $\mu$V of the N20 response has a sensitivity of 90.1%, a specificity of 64,5%, a PPV of 65% and a NPV of 72.7%; and a classifier with an absolute N20 amplitude threshold value between 0.25 and 0.5 $\mu$V of the N20 response has a sensitivity of between 86-88%, a specificity between 62-64%, a PPV value of 65% and NPV value of between 72-76%. For an N20 SEP absolute amplitude threshold comprised between 0.25 and 0.5, $\mu$V an optimum sensitivity and acceptable specificity is obtained for a functional prognostic before an endovascular stroke treatment at 90 days. The most optimal sensitivity and specificity is obtained for an N20 SEP absolute amplitude threshold is comprised between 0.33-0.35 $\mu$V.

2.3.2 Functional prognostic (shift analysis) at 90 days

**Post-hoc analysis**

Patients with $N_{20}$ present have an overall better functional prognosis in all categories of the mRS scale. Seventy-two percent of patients with present $N_{20}$ response had a score of 0-2 on the mRS scale at 90 days post-stroke, while this was only 14.3% in the group of patients with baseline $N_{20}$ absent. Notably, the percentage of patients with mRS scores of 4 and 5-6 is clearly lower in patients with $N_{20}$ present (10.9 vs 25% and 15.8 vs 51.8%, respectively).

**Univariate analysis**

When relating the clinical variables assessed in the study to the functional prognosis according to all degrees of disability at 90 days, it was observed that the variables related to $N_{20}$, especially the dichotomised $N_{20}$ amplitude, have a better capacity to predict an optimal functional prognosis than other variables used in routine clinical practice such as the NIHSS or ASPECTS scales (Table 21).

*Table 21: Univariate analysis. Predictive capacity of functional prognosis according to all degrees of disability at 90 days of the clinical variables. The OR and Brier coefficient (low values indicate better predictive capacity) are shown for the clinical variables of the study. The variables are ordered according to the Brier coefficient. \*All neurophysiological variables refer to the cerebral hemisphere affected by the stroke. rTPA: intravenous fibrinolysis. #N20 latency has been corrected to avoid the effect of the value "0".*

| Clinical variables | Raw OR | 95% IC | Brier | p |
|---|---|---|---|---|
| N20 "discr" Latency * (per unit, ms) | 0.89 | 0.85-0.92 | 0.178 | **<0.001** |
| Presence N20 (yes)* | 9.91 | 5,19-19,43 | 0.190 | **<0.001** |
| Amplitude logN20 | 1.73 | 1.42-2.11 | 0.197 | **<0.001** |
| NIHSS | 0.87 | 0.82-0.91 | 0.223 | **<0.001** |
| Amplitude N20 (per unit, $\mu$V)* | 1,19 | 1,06-1,34 | 0,237 | **0,002** |
| ASPECTS | 1,38 | 1,17-1,64 | 0,239 | **<0,001** |
| Age | 0,97 | 0,95-0,99 | 0,234 | **0,004** |
| Glycaemia | 0,99 | 0,98-0,99 | 0,236 | **<0,001** |
| Mean arterial pressure (MAP) | 0,99 | 0,97-1,01 | 0,248 | 0,204 |
| rTPA+TM | 1,61 | 0,99-2,64 | 0,244 | 0,055 |
| Left laterality (yes) | 0,82 | 0,50-1,34 | 0,250 | 0,428 |
| Sex (woman) | 1 | 0,61-1,64 | 0,250 | 0,999 |
| Occlusion ACM-M1 | 1,30 | 0,67-2,53 | 0,246 | 0,445 |
| Occlusion ACM-M2 | 2,03 | 0,83-4,98 | 0,246 | 0,445 |
| Occlusion-tandem | 1,53 | 0,70-3,34 | 0,246 | 0,445 |

**Multivariate analysis**

A similar multivariate analysis was performed defining models A and B as in section 2.3.1. The multivariate analysis showed that the baseline variables associated with the ordinal mRS at 90 days are age (older age, worse outcome), baseline NIHSS (higher baseline severity, worse outcome), blood glucose (higher values, worse outcome), ASPECTS (higher values associated with better outcome) and baseline amplitude or presence of the $N_{20}$ response (values present are associated with better outcome than absent values); being the presence the one with the best functional prognosis (OR 6.67 model A and OR 7.14 model B) (see Table 22).

*Table 22: Multivariate analysis: clinical variables and functional prognosis according to all degrees of disability 90 days after stroke. \*All neurophysiological variables refer to the cerebral hemisphere affected by the stroke. Baseline amplitude was only included in one modality (quantitative, log-transformed and categorized), depending on whether it provided a lower Brier score. rTPA: intravenous fibrinolysis.*

| | Model A | | Model B | |
|---|---|---|---|---|
| | OR (IC 95%) | p | OR (IC 95%) | p |
| Age | 0.97 (0.94-0.99) | **0,010** | 0.97 (0.94-0.99) | **0.004** |
| NIHSS | 0,89 (0,83-0,95) | **<0,001** | 0,91 (0,86-0,96) | **0,001** |
| rTPA | 2,07 (1,08-4,01) | **0,029** | 1,39 (0,81-2,40) | 0,237 |
| Glycaemia (per unit, mg/dl) | 0,99 (0,98-1) | 0,242 | 0,99 (0,98-1) | 0,069 |
| ASPECTS | 1,44 (1,15-1,84) | **0,002** | 1,21 (1-1,47) | 0,056 |
| Amplitude logN20*# | 1,52 (1,22-1,92) | **<0,001** | | |
| Presence N20 (yes)* | | | 6,84 (3,24-14,79) | **<0,001** |

**2.4 Prognostic capability at 7 days of the N20 response before the MT with neuroimaging tests**

Finally, the performance of several models including the categorical N20 response (present/absent) and one or more neuroimaging tests was assessed. On their own, each assessment technique have different odds ratio (OR), predictive capability (AUC) and statistical significance (p) as seen in Table 23.

*Table 23 Univariate analysis of N20 (categorical) and imaging variables. *Patients with non-assessable N20 were not considered. The ASPECTS scale was considered as a continuous variable.*

| Imaging test variable | N | OR (95% IC) | AUC | p |
|---|---|---|---|---|
| **Collateral circulation state** | 191 | 2.58 (1.38-4.97) | 0.612 | 0.004 |
| **Ischemia core** | 127 | 0.97 (0.95-0.99) | 0.657 | 0.006 |
| **Tmax>6** | 116 | 1.00 (0.99-1.00) | 0.541 | 0.364 |
| *HIR* | 111 | 0.54 (0.10-2.72) | 0.548 | 0.455 |
| **ASPECTS Scale** | 199 | 1.48 (1.19-1.89) | 0.636 | 0.001 |
| **N20*** | 168 | 14.00 (5.28-48.57) | 0.713 | 0.000 |

The categorical N20 response has a better prognostic capability than any other imaging test variable in the study. It provided a significant improvement against the best neuroimaging test predictor, the ischaemia core score (AUC 0.713 vs 0.657).

In the multivariate analysis, the univariate models were adjusted to only include patients with valuable N20 response. Tmax >6 and HIR variables were excluded for their low performance. Five models were adjusted:

- Model 0: Categorical N20 response (N20)
- Model 1: Collateral circulation+N20
- Model 2: Core ischaemic lesion+N20
- Model 3: ASPECTS scale score+N20
- Model 4: Collateral circulation status + core ischemic injury + ASPECTS scale score + N20

As it can be seen, the categorical N20 response alone, its combination with the collateral circulation, core ischaemic lesion, and ASPECTS scale score independently, and altogether have been used to build these models. The OR, for each variable within each model and the AUC for each model is summarised in Table 24.

*Table 24: Multivariate analysis. Adjusted predictive ability of N20 response (categorical) and imaging variables. *Patients with non-assessable N20 were not considered. The variables Tmax>6 and HIR were excluded.*

| Model | N | Collateral circulation OR (95%IC) | *Core* OR (95%IC) | ASPECTS OR (95%IC) | N20 OR (95% IC) | AUC |
|---|---|---|---|---|---|---|
| Model 0 | 168 | | | | 14 (5.28-48.57) | 0.713 |
| Model 1 | 191 | 2.20 (1.10-4.50) | | | 13.10 (4.33 -57) | 0.739 |
| Model 2 | 127 | | 0.97 (0.94-0.99) | | 15.23 (4.00-100.75) | 0.774 |
| Model 3 | 199 | | | 1.42 (1.12-1.85) | 13.07 (4.31-56.96) | 0.752 |
| Model 4 | 124 | 2.94 (1.10-8.40) | 0.98 (0.95-1.01) | 1.17 (0.74-1.87) | 15.33 (3.92-102.97) | 0.813 |

The combination of the categorical N20 response (absent//present) with neuroimaging tests provided better prediction capability than current multimodal/advanced neuroimaging techniques. Also, the combination of all tests with the categorical N20 response provided the best prediction capability with an AUC of 0.813. It is to be noted that the categorical N20 response improved the neuroimaging tests AUC in 0.12 points when included as a prognosis tool.

These results show that the categorical N20 response not only provides a better prognostic capability that known techniques, but it also significantly improves the prognostic capability of these techniques (collateral circulation, ischemic core and ASPECTS scale score). The best prognostic capability is achieved with the combination of all the neuroimaging tests scores and the categorical N20 response variable.

## 2.5 Prognostic capability at 7 days of the N20 response after the MT

2.5.1 Good functional prognostic (mRS ≤ 2) at 7 days

**Post-hoc analysis**

For the post treatment analysis the following variables were tested: TICI scale at the end of mechanical thrombectomy, the duration of ischaemia (time between the onset of symptoms and the time of arterial recanalisation), the presence of complications during the procedure and of general complications after stroke (in the first 72 hours and after this time limit). Patients with a good functional prognosis (mRS≤2) had a higher percentage of optimal arterial recanalisation (TICI scale ≥2b) and fewer overall post-stroke complications, both early (first 72 hours) and late (beyond the first 72 hours) (see Table 25).

*Table 25: Final clinical and time flow variables in patients with good (mRS≤2) and poor (mRS>2) functional prognosis at 7 days. Optimal functional prognosis is defined as a score ≤2 on the mRS scale. TM: mechanical thrombectomy; TICI: Thrombolysis in Cerebral Infarction. \*Chi-square.*

| Clinical and time variables (N=223) | mRS ≤2 (n=77, 48%) | mRS>2 (n=146, 52%) | p |
|---|---|---|---|
| TICI final scale, n(%)<br>0<br>1<br>2a<br>2b<br>2c<br>3 | 1 (1.3)<br>0<br>0(2)<br>17 (22.1)<br>8 (10.4)<br>51 (66.2) | 22(15.1)<br>2(1.4)<br>13(8.9)<br>35(24)<br>17 (11.6)<br>57 (39) | **<0.001\*** |
| Ichaemia duration (minutes)<br>Mean (SD) | 377.9 (209.7) N=76 | 464.3 (317.6) N=121 | **0.037** |
| Complications during *MT* ( yes )<br>n(%) | 12 (15.6) | 28 (19.2) | 0.21 |
| Complications <72h ( yes )<br>n(%) | 7 (9.1) | 60 (41.1) | **0.006** |
| Complications>72 h ( yes )<br>n(%) | 3 (3.9) | 36 (24.7) | **0.046** |

**Univariate analysis**

When relating the clinical variables considered relevant during and after mechanical thrombectomy with an optimal functional prognosis 7 days after stroke, it was observed that the variables related to N20 in the pathological cerebral hemisphere at the end of the procedure, especially the amplitude and latency of N20 in absolute value, have a better capacity to predict an optimal functional prognosis than the rest of the variables studied (Table 26).

*Table 26: Univariate analysis. Predictive capacity of post-MT clinical variables for optimal functional prognosis at 7 days. \*All neurophysiological variables refer to the cerebral hemisphere affected by the stroke.*

| Clinical variables | Raw OR | 95% CI | AUC | p |
|---|---|---|---|---|
| Amplitude N20* (μV) | 1.83 | 1.42-2.42 | 0.823 | **<0.001** |
| Amplitude logN20* | 3.07 | 2.21-4.57 | 0.823 | **<0.001** |
| Latency N20* (ms) | 0.77 | 0.67-0.84 | 0.811 | **<0.001** |

(continued)

| Clinical variables | Raw OR | 95% CI | AUC | p |
|---|---|---|---|---|
| Presence N20* (yes) | 187.82 | 11.06-3188.57 | 0.795 | <0.001 |
| Complications (<72h) (yes) | 0.14 | 0.06-0.31 | 0.660 | **<0.001** |
| TICI | 25.80 | 5.40-462.98 | 0.620 | **<0.001** |
| Complications (>72h) (yes) | 0.124 | 0.029-0.360 | 0.604 | **<0.001** |
| Ischemia duration (min) | 0.999 | 0.998-1.000 | 0.558 | **0.028** |
| Complications during *MT* (yes) | 0.768 | 0.360-1.601 | 0.518 | 0.502 |

**Multivariate analysis**

A multivariate analysis was performed. In multivariate model B all significant variables with p<0.1 were included, including the (present/absent) amplitude of $N_{20}$ was used while in the multivariate model A, the quantitative log-amplitude of $N_{20}$ and the 2 variables that are significant at the univariate level, but not at the multivariate level were included: medical complications in the first 72 hours and the duration of ischaemia.. Multivariate analysis showed that in both models categorical (dichotomic) $N_{20}$ response amplitude behaved as an independent factor associated with good functional prognosis at 7 days. (Table 27).

*Table 27: Multivariate analysis. Predictive capacity of 7-day optimal functional prognosis of clinical variables post-mechanical thrombectomy *All neurophysiological variables refer to the cerebral hemisphere affected by the stroke.*

| | Model A | | Model B | |
|---|---|---|---|---|
| | OR (IC 95%) | p | OR (IC 95%) | p |
| Medical complications (<72h) (yes) | 0.84 (0.23-3.05) | 0.795 | 0.44 (0.08-2.00) | **0.014** |
| Medical complications (>72 h) (yes) | 0.09 (0.00-0.69) | **0.043** | 0.96 (0.19-5.02) | 0.336 |
| Ischaemia duration | 1.00 (1.00-1.00) | 0.233 | 1.00 (1.00-1.00) | 0.077 |
| Amplitude logN20* | 2.70 (1.90-4.29) | **<0.001** | | |
| Presence N20 (yes)* | | | 21.77 (0.77-inf) | 0.983 |

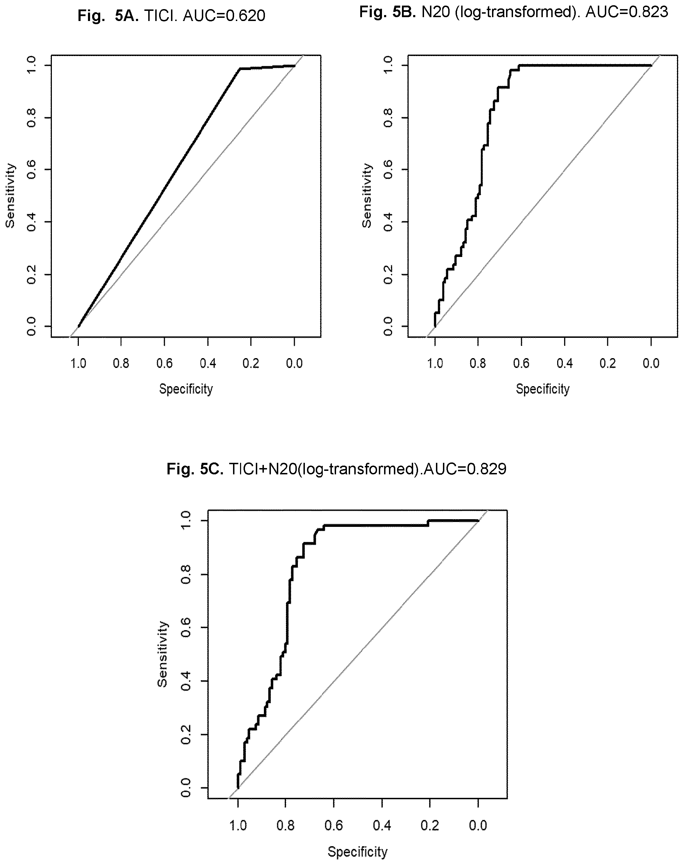

Logistic regression models have also been constructed for different clinical variables of interest in isolation or in combination: final TICI scale, post-intervention N20 amplitude (quantitative and log-transformed) and the combination of the two (see Figure 5 A-C for the models ROC curves and Table 28 for the models regressions AUC):

- Model 0a: final TICI (scale determining the degree of arterial recanalisation)
- Model 1: N20 post-intervention amplitude (quantitative and log-transformed)
- Model 2: Final TICI+N20 post-intervention amplitude (quantitative and log transformed)

*Table 28: Models logistic regression for 7-day prediction after the MT: number of patients and AUC values.*

| | N | AUC |
|---|---|---|
| Model 0 | 223 | 0.620 |
| Model 1 | 165 | 0.823 |
| Model 2 | 165 | 0.829 |

The $N_{20}$ amplitude alone (Model 1) still has a higher predictive ability for good functional prognosis 7 days after stroke than the TICI scale (Model 0) (AUC 0.823 vs. AUC 0.620). The combination of both slightly increases the prediction capability of N20 amplitude alone (AUC 0.829 vs 0.823.

A further analysis of the model 1 was performed to determine the current amplitude values that better determine the difference between a good and bad outcome, i.e. the values that provide the best determination thresholds. Several threshold cuts were made and the sensitivity specificity, PPV and NPV were calculated for each of them (see Table 29).

**Table 29:** Classification performance post-intervention at 7 days for different threshold cuts.

| Cut | Prob | Sensitivity Mean (95% CI) | Specificity Mean (95% CI) | PPV Mean (95% CI) | NPV Mean (95% CI) |
|---|---|---|---|---|---|
| 0 | 0.21 | 1.00 (1.00-1.00) | 0.63 (0.53-0.73) | 0.36 (1.00-0.36) | NaN (1.00-NaN) |
| 0.25 | 0.28 | 1.00 (0.86-1.00) | 0.64 (0.55-0.75) | 0.53 (0.98-0.59) | |
| 0.5 | 0.37 | 0.98 (0.81-1.00) | 0.64 (0.55-0.75) | 0.55 | |
| 0.75 | 0.48 | 0.88 (0.53-1.00) | 0.73 (0.62-0.81) | 0.56 | |
| 1 | 0.58 | 0.81 (0.41-0.97) | 0.74 (0.64-0.82) | 0.57 | 0.83 (0.83-0.89) |
| 1.25 | 0.68 | 0.69 (0.36-0.93) | 0.77 (0.69-0.85) | 0.53 | 0.75 (0.68-0.81) |
| 1.5 | 0.76 | 0.49 (0.27-0.88) | 0.81 (0.74-0.90) | 0.47 (0.47-0.58) | 0.68 (0.47-0.74) |
| 1.75 | 0.83 | 0.44 (0.22-0.80) | 0.84 (0.75-0.92) | 0.44 (0.41-0.57) | 0.67 (0.41-0.72) |
| 2 | 0.88 | 0.39 (0.20-0.71) | 0.84 (0.75-0.92) | 0.47 (0.41-0.60) | 0.67 (0.41-0.72) |
| 2.25 | 0.92 | 0.36 (0.19-0.64) | 0.85 (0.76-0.92) | 0.46 (0.37-0.59) | 0.67 (0.37-0.71) |
| 2.5 | 0.94 | 0.32 (0.17-0.54) | 0.90 (0.81-0.97) | 0.39 (0.27-0.55) | 0.65 (0.27-0.68) |
| 2.75 | 0.96 | 0.25 (0.14-0.46) | 0.90 (0.81-0.97) | 0.44 (0.27-0.62) | 0.65 (0.27-0.69) |
| 3 | 0.97 | 0.25 (0.14-0.46) | 0.92 (0.82-0.98) | 0.40 (0.24-0.58) | 0.65 (0.24-0.68) |
| 3.5 | 0.99 | 0.20 (0.07-0.36) | 0.94 (0.84-0.99) | 0.46 (0.20-0.67) | 0.65 (0.20-0.68) |
| 4 | 1 | 0.15 (0.03-0.32) | 0.96 (0.91-1.00) | 0.41 (0.14-0.67) | 0.64 (0.14-0.67) |
| 7 | 1 | 0.05 (0.00-0.15) | 1.00 (0.95-1.00) | 1.00 (0.05-1.00) | 0.64 (0.05-0.65) |

It was observed that a classifier with a log-transformed N20 amplitude threshold value between 0.25 and 0.5 $\mu$V of the N20 response has a sensitivity between 98% and 100% (95% CI 86%-100%), a specificity of 64%, a PPV between 53 and 55% and a NPV of 96%.

For an N20 SEP absolute amplitude threshold comprised between 0.25 and 0.5, $\mu$V an optimum sensitivity and acceptable specificity is obtained for a functional prognostic after an endovascular stroke treatment at 7 days.

2.5.2 Functional prognostic (shift analysis) at 7 days

**Post-hoc analysis**

Patients with N20 present have an overall better functional prognosis in all categories. Notably, no patient with no N20 response at the end of MT had an mRS score between 0-2 at 7 days post-stroke. Also, the percentage of patients with mRS scores of 5 and 6 (major dependents and successes) is clearly higher in patients with absent N20 (17 vs. 4% and 11 vs. 0.4%, respectively).

**Univariate analysis**

When relating the clinical variables assessed in the study to the functional prognosis according to all degrees of disability at 7 days, it was observed that the variables related to $N_{20}$, especially the latency and the log-transformed $N_{20}$ amplitude, have a better capacity to predict an optimal functional prognosis than the arterial recanalization itself (TICI scale) or post-procedural medical complications (Table 30).

*Table 30: Univariate analysis. Predictive capacity of functional prognosis according to all degrees of disability at 7 days of post-MT clinical variables. OR and Brier coefficient are shown (low values indicate better predictive ability). Variables are ordered according to the Brier coefficient. *All neurophysiological variables refer to the cerebral hemisphere affected by the stroke. #The latency of N20 has been corrected to avoid the effect of the value "0".*

| Clinical variables | Raw OR | 95% CI | Brier | p |
|---|---|---|---|---|
| N20* Latency (ms)# | 0.820 | 0.783-0.856 | 0.152 | **<0.001** |
| Amplitude logN20* | 3.078 | 2.422-3.980 | 0.157 | **<0.001** |
| N20 Presence (yes)* | 31.329 | 3.846-15.340 | 0.165 | **<0.001** |
| N20 Amplitude (por unidad, $\mu$V)* | 2.017 | 1.630-2.544 | 0.191 | **<0.001** |
| Complications (<72h) (yes) | 0.105 | 0.055-0.191 | 0.201 | **<0.001** |
| Final TICI | 11.315 | 5.159-27.716 | 0.205 | **<0.001** |
| Complications (>72h) (yes) | 0.107 | 0.045-0.228 | 0.211 | **<0.001** |
| Complications MT | 0.796 | 0.430-1.459 | 0.226 | 0.461 |
| Ischemia duration (min) | 0.999 | 0.999-1.000 | 0.233 | 0.133 |

**Multivariate analysis**

A similar multivariate analysis was performed as in the good functional prognostic analysis, including all significant variables with p<0.1 in model A (including the log-transformed amplitude of $N_{20}$ ($\mu$V)) and model B (including the categoric (present/absent) amplitude of $N_{20}$ (%)) were designed.

The multivariate analysis showed that the baseline variables associated with the ordinal mRS at 7 days are complications beyond 72 hours after stroke (more complications, worse outcome) and the extent (log-transformation) or presence of the N20 response (higher values or presence associated with better outcome) (Table 31)

*Table 31: Multivariate analysis: clinical variables and functional prognosis according to all degrees of disability 7 days after stroke. *All neurophysiological variables refer to the cerebral hemisphere affected by the stroke. Baseline amplitude was only included in one modality (quantitative, log-transformed and categorised), depending on whether it provided a lower Brier score.*

| | Model A | | Model B | |
|---|---|---|---|---|
| | OR (CI 95%) | p | OR (CI 95%) | p |
| Complications (<72h) (yes) | 0.57 (0.24-1.35) | 0.208 | 0.21 (0.09-0.45) | **<0.001** |
| Complications (>72h) (yes) | 0.08 (0.02-0.31 | **0.001** | 0.42 (0.14-1.18 | 0.107 |
| Amplitude logN20* | 2.85 (2.21-3.74) | **<0.001** | | |
| Presence N20 (yes)* | | | 23.92 (11.30-53.17) | **<0.001** |

**2.6 Prognostic capability at 90 days of the N20 response after the MT**

2.6.1 Good functional prognostic (mRS $\leq$ 2) at 90 days

**Post-hoc analysis**

The mRS was evaluated at 90 days for 204 patients .Good functional prognostic was observed in 98 patients (48%). They were younger, had inferior glucose and diastolic arterial tension levels, a lower initial score in the NIHSS scale and a smaller ischemic tissue extension (Table 32)

*Table 32: Final clinical and time flow variables in patients with good (mRS≤2) and poor (mRS>2) functional prognosis 90 days after stroke. Optimal functional prognosis is defined as a score ≤2 on the mRS scale. MT: Mechanical thrombectomy; TICI: Thrombolysis in Cerebral Infarction. &Mann-Whitney U; ¶Non-parametric test for median comparison; #t-Student. In the case of the variable "N20 latency" in the pathological cerebral hemisphere, the value "0" has not been included when N20 was absent. \*"Discr." Latency: the variable "discriminant latency" has also been created to obviate the effect of the "0" values when the N20 response is absent. It consists of the difference in absolute value with respect to the normal latency of the N20 response (20±5 ms). In the variable "amplitude N20 pathological cerebral hemisphere", the value "0" has been included when the N20 response was absent.*

| Clinical and time variables (N=204) | mRS ≤2 (n=98, 48%) | mRS>2 (n=106, 52%) | p |
|---|---|---|---|
| TICI final scale, n(%) | | | |
| 0 | 2 (2) | 19 (17.9) | |
| 1 | 0 | 1 (0.9) | **<0.001** |
| 2a | 2 (2) | 11 (10.4) | |
| 2b | 23 (23.5) | 24 (22.6) | |
| 2c | 12 (12.2) | 11 (10.4) | |
| 3 | 59 (60.2) | 40 (37.7) | |
| Ichaemia duration (minutes) | | | |
| Mean (SD) | 382,52 (225,69) n=96 | 485,80 (315,11) n=104 | **0.031** |
| Complications duringMT (yes) | | | |
| n(%) | 12 (12.2) | 20(18.9) | 0.10 |
| Complications <72h ( yes ) | | | |
| n(%) | 12 (12.2) | 52 (49.1) | **<0.001** |
| Complications>72 h ( yes ) | | | |
| n(%) | 5 (5.1) | 32 (30.2) | **0.003** |
| Presence N20 (yes) | | | |
| n(%) | 79 (86,8) | 12 (13,2) | **<0,001** |
| N20 Amplitude# (ms) | | | |
| Mean (SD) | 2,3 (2,6) | 0,3 (0,9) | **<0,001&** |
| Median (Quartiles) | 1,4 (1,01-2,5) | 0 (0-0) | **<0,001¶** |
| N20 Latency (ms) | | | |
| Mean (SD) Median (Quartiles) | 22,4 (11,04) 20,8 (19,3-22,7) | 21,5 (2,6) 22,02 (19,4-23,6) | 0,777" 0,293¶ |
| N20 "discr" Latency\* (ms) | | | |
| Mean (SD) | 3,5 (10,9) | 17,1 (6,5) | **<0,001&** |
| Median (Quartiles) | 1,6 (0,7-2,9) | 20 (20-20) | **<0,001¶** |

## Univariate analysis

When relating the relevant post MT clinical variables considered in the study to an optimal functional prognosis 90 days after stroke, it was equally observed N20-related variables, especially latency and amplitude, both absolute and dichotomised, were found to have a better ability to predict optimal functional prognosis than either the TICI score at the end of the procedure or the duration of ischaemia time. (Table 33). It is noted the predictive power of the dichotomised $N_{20}$ amplitude (OR = 500) and the log transformed $N_{20}$ amplitude (OR=5.85).

*Table 33: Univariate analysis. Predictive capacity of post-intervention clinical variables for optimal functional prognosis at 90 days. All neurophysiological variables refer to the cerebral hemisphere affected by the stroke. Variables are ordered from highest to lowest according to their predictive ability expressed by the area under the curve (AUC). \*"Discr." Latency: the variable "discriminant latency" has also been created to obviate the effect of the "0" values when the N20 response is absent. It consists of the difference in absolute value with respect to the normal latency of the N20 response (20±5 ms). In the variable "amplitude N20 pathological cerebral hemisphere", the value "0" has been included when the N20 response was absent. ≠For the variable "N20 amplitude" and "logN20 amplitude" in the pathological cerebral hemisphere, the value "0" has been included when the N20 response was absent. #Neurological: progressive stroke, malignant infarction, stroke recurrence, comorbid seizure, asymptomatic IH1 and IH2 haemorrhagic transformation, symptomatic intracranial haemorrhage, hyperperfusion syndrome, coma. Systemic: systemic haemorrhage, atrial fibrillation, hypertensive crisis, hypotension, chest pain, other cardiovascular complications, aspiration pneumonia, systemic infection, cardiorespiratory arrest, respiratory infection, peripheral embolism, acute urinary retention, anaemia, haematological complications, angioedema, agitation. ±Arterial rupture with contrast extravasation, arterial dissection, distal embolism, subarachnoid haemorrhage, vasospasm requiring treatment, haemodynamic complications (hypertension, hypotension, bradycardia), device rupture, reocclusion, femoral thrombosis.*

| Clinical variables | Raw OR | 95% IC | AUC | p |
|---|---|---|---|---|
| N20 Latency * (ms) | 0.73 | 0.64-0.79 | 0.935 | **<0.001** |
| N20 Amplitude≠ ($\mu$V) | 5.29 | 3.125-9.80 | 0.918 | **<0.001** |
| logN20 Amplitude≠ | 5.84 | 3.82-10 | 0.918 | **<0.001** |
| N20 Presence (yes) | 414,75 | 76,97-7672,67 | 0,900 | **<0,001** |
| Complications (<72h) (yes) | 0.14 | 0.07-0.29 | 0.684 | **<0.001** |
| TICI | 9.71 | 3.65-33.3 | 0.626 | **<0.001** |
| Complications (>72h) (yes) | 0.12 | 0.04-0.31 | 0.625 | **<0.001** |
| Ischemia duration¥ (min) | 0.99 | 0.99-1 | 0.576 | **0.026** |
| Complications during $MT^\pm$ (yes) | 0.60 | 0.27-1.29 | 0.533 | 0.191 |

## Multivariate analysis

A multivariate analysis was performed. In multivariate model A all significant variables with p<0.1 were included, "complications within 72 hours of stroke", which was significant in the univariate analysis, but not significant in the multivariate analysis; while in the multivariate model B an automatic variable selection process (AIC) was used. Multivariate analysis showed that in both models, post-procedural variables associated with functional prognosis at 90 days are the log-transformed amplitude of the N20 response in the stroke-affected hemisphere at the end of the procedure and complications beyond 72 hours after stroke onset (Table 34).

*Table 34: Multivariate analysis. Predictive capacity of post-intervention clinical variables for optimal functional prognosis at 90 days. \*All neurophysiological variables refer to the cerebral hemisphere affected by the stroke.*

| | Model A | | Model B | |
|---|---|---|---|---|
| | OR (CI 95%) | p | OR (CI 95%) | p |
| Complications <72h (yes) | 0.91 (0.17-4.32) | 0.904 | | |
| Complications >72h (yes) | 22.26 (2.60-233.95) | **0.006** | 20.80 (3.30-164.40) | **0.002** |
| Ischemia duration (minutes) | 1.00 (1.00-1.00) | 0.130 | 1.00 (1.00-1.00) | 0.123 |
| logN20 Amplitude * | 0.18 (0.09-0.29) | **<0.001** | 0.18 (0.09-0.29) | **<0.001** |

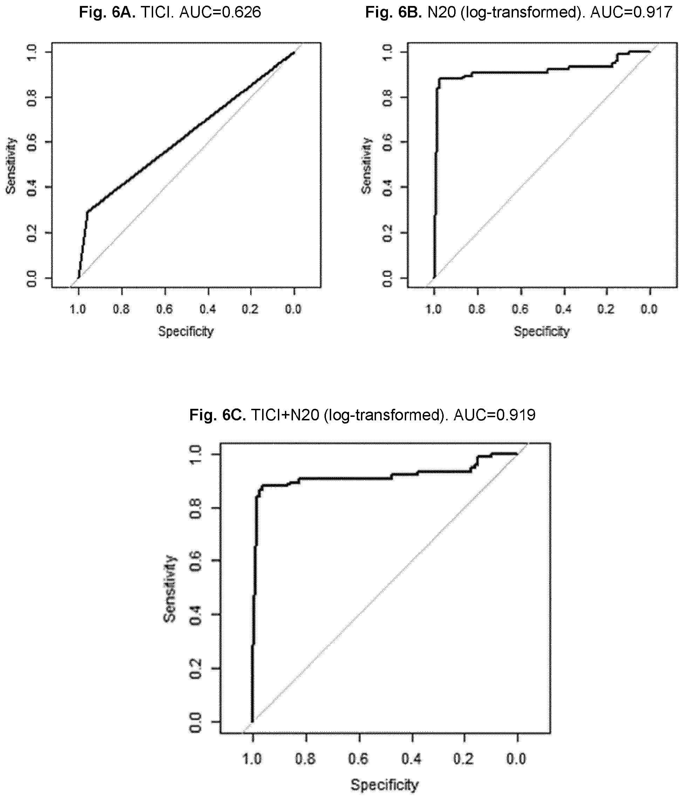

Logistic regression models have also been constructed for different clinical variables of interest in isolation or in combination: Final ICTI and Post-intervention quantitative and transformed N20 (See figure 6 A-C for the models ROC curves and Table 35 for the models regressions AUC):
- Model 0: Final TICI
- Model 1: post-intervention N20 amplitude (quantitative and log-transformed)
- Model 2: final TICI and post-intervention N20 amplitude(quantitative and log-transformed)

*Table 35: Models logistic regression for 90-day prediction after the MT: number of patients and AUC values.*

|  | N | AUC |
|---|---|---|
| Modelo 0 | 204 | 0.626 |
| Modelo 1 | 155 | 0.917 |
| Modelo 2 | 155 | 0.919 |

The $N_{20}$ response alone (Model 1) still has a higher predictive ability for good functional prognosis 90 days after stroke than the TICI scale (Model 0) (AUC 0.917 vs. AUC 0.626). As in the 7-day outcome analysis, adding N20 to TICI scale significantly increases their predictive ability, especially when N20 is measured qualitatively or dichotomously (presence/absence) (Model 2).

A further analysis of the model 1 was performed to determine the current amplitude values that better determine the difference between a good and bad outcome, i.e. the values that provide the best determination thresholds. Several threshold cuts were made and the sensitivity specificity, PPV and NPV were calculated for each of them (see Table 36).

**Table 36:** Classification performance post-intervention at 90 days for different threshold cuts.

| Cut | Prob | Sensitivity Mean (95% CI) | Specificity Mean (95% CI) | PPV Mean (95% CI) | NPV Mean (95% CI) |
|---|---|---|---|---|---|
| 0 | 0.21 | 1.00 (1.00-1.00) | 0.00 (0.00-0.92) | 0.52 | NaN (1.00-NaN) |
| 0.25 | 0.28 | 0.98 (0.47-1.00) | 0.87 (0.52-0.95) | 0.82 | 0.93 |
| 0.5 | 0.37 | 0.96 (0.22-1.00) | 0.88 (0.76-0.95) | 0.84 | 0.91 |
| 0.75 | 0.48 | 0.82 (0.12-1.00) | 0.91 (0.83-0.96) | 0.84 | 0.76 |
| 1 | 0.58 | 0.82 (0.12-1.00) | 0.91 (0.84-0.96) | 0.83 (0.78-0.90) | 0.72 |
| 1.25 | 0.68 | 0.82 (0.12-1.00) | 0.91 (0.84-0.96) | 0.80 (0.62-0.88) | 0.63 (0.62-0.69) |
| 1.5 | 0.76 | 0.49 (0.11-1.00) | 0.92 (0.85-0.97) | 0.75 (0.44-0.85) | 0.56 (0.44-0.61) |
| 1.75 | 0.83 | 0.49 (0.11-1.00) | 0.93 (0.87-0.97) | 0.72 (0.38-0.83) | 0.54 (0.38-0.58) |
| 2 | 0.88 | 0.39 (0.10-0.99) | 0.93 (0.87-0.99) | 0.74 (0.36-0.85) | 0.54 (0.36-0.58) |
| 2.25 | 0.92 | 0.39 (0.10-0.99) | 0.93 (0.87-0.99) | 0.72 (0.32-0.84) | 0.52 (0.32-0.56) |
| 2.5 | 0.94 | 0.39 (0.10-0.99) | 0.93 (0.88-0.99) | 0.65 (0.25-0.80) | 0.50 (0.25-0.54) |
| 2.75 | 0.96 | 0.39 (0.10-0.99) | 0.95 (0.88-1.00) | 0.61 (0.21-0.77) | 0.49 (0.21-0.53) |
| 3 | 0.97 | 0.39 (0.10-0.99) | 0.95 (0.88-1.00) | 0.57 (0.19-0.75) | 0.49 (0.19-0.52) |
| 3.5 | 0.99 | 0.17 (0.07-0.89) | 0.97 (0.89-1.00) | 0.60 (0.15-0.80) | 0.49 (0.15-0.51) |
| 4 | 1 | 0.14 (0.05-0.34) | 1.00 (0.92-1.00) | 0.72 (0.11-0.90) | 0.49 (0.11-0.51) |
| 7 | 1 | 0.11 (0.05-0.22) | 1.00 (1.00-1.00) | 1.00 (0.04-1.00) | 0.48 (0.04-0.49) |

It was observed that a classifier with a log-transformed N20 amplitude threshold value between 0.25 and 0.5 $\mu$V of the N20 response has a sensitivity between 96% and 98%, a specificity between 87 and 88%, a PPV between 82 and 84% and a NPV between 91 and 93%.

For an N20 SEP absolute amplitude threshold comprised between 0.25 and 0.5, $\mu$V an optimum sensitivity and acceptable specificity is obtained for a functional prognostic after an endovascular stroke treatment at 90 days.

2.6.2 Functional prognostic (shift analysis) at 90 days

**Post-hoc analysis**

Patients with $N_{20}$ present have an overall better functional prognosis in all categories of the mRS scale. Only one patient with no N20 response at the end of MT had an mRS score of 1 at 90 days post-stroke. Also, the percentage of patients with mRS scores of 5 and 6 (major dependents and exits) is clearly higher in patients with absent N20 (14.1 vs 0% and 45.3 vs 4.4%, respectively).

**Univariate analysis**

When relating the clinical variables assessed in the study to the functional prognosis according to all degrees of disability at 90 days, it was observed that the variables related to $N_{20}$, specially N20 latency and amplitude (logarithmic and qualitative transformation), have a better ability to predict functional prognosis with respect to arterial recanalisation itself (TICI scale) or post-procedural medical complications.(Table 37)

*Table 37: Univariate analysis. Predictive capacity of functional prognosis according to all degrees of disability at 90 days of post-MT clinical variables. ORs and Brier coefficient are shown (low values indicate better predictive ability). Variables are ordered according to the Brier coefficient. *All neurophysiological variables refer to the cerebral hemisphere affected by the stroke. #The latency of N20 has been corrected to avoid the effect of the value "0".*

| Clinical variables | Raw OR | 95% CI | Brier score | p |
|---|---|---|---|---|
| N20 Latency * (ms) | 0.79 | 0.75-0.83 | 0.078 | **<0.001** |
| logN20 Amplitude* | 3.28 | 2.52-4.35 | 0.087 | **<0.001** |
| N20 Presence * (yes) | 50 | 20-111.1 | 0.115 | **<0.001** |
| N20 Amplitude* ($\mu$V) | 1.54 | 1.29-1.87 | 0.187 | **<0.001** |
| Complications (<72h) (yes) | 0.12 | 0.07-0.22 | 0.211 | **<0.001** |
| TICI | 6.58 | 3.30-13.70 | 0.222 | **<0.001** |
| Complications (>72h) (yes) | 0.11 | 0.05-0.22 | 0.223 | **<0.001** |
| Ischemia duration (min) | 0.99 | 0.99-1 | 0.243 | **0.018** |
| Complications during *MT* (yes) | 0.78 | 0.40-1.50 | 0.248 | 0.452 |

**Multivariate analysis**

A similar multivariate analysis was performed defining models A and B as in section 2.5.2. The multivariate analysis showed that the baseline variables associated with the ordinal mRS at 90 days are the log-transformed amplitude of the N20 response post-MT (higher values are associated with better outcome) and medical complications after stroke (both within and beyond 72 hours of symptom onset) (the more complications, the worse the evolution). Although not significant, the duration of ischaemia were retained in both models as an adjustment variable. ( see Table 38)

*Table 38: Multivariate analysis. Predictive capacity of post-intervention clinical variables for functional prognosis at 90 days. *All neurophysiological variables refer to the cerebral hemisphere affected by the stroke.*

| | Model A | | Model B | |
|---|---|---|---|---|
| | OR (CI 95%) | p | OR (CI 95%) | p |
| Complications <72h (yes) | 0.46 (0.18-1.15) | **0.096** | 0.46 (0.18-1.15) | **0.096** |
| Complications >72h (yes) | 0.20 (0.06-0.69) | **0.011** | 0.20 (0.06-0.69) | **0.011** |
| Ischemia duration (minutes) | 1 (1.00-1.00) | 0.156 | 1 (1.00-1.00) | 0.156 |

(continued)

| | Model A | | Model B | |
|---|---|---|---|---|
| | OR (CI 95%) | p | OR (CI 95%) | p |
| logN20 Amplitude * | 2.78 (2.08-3.70) | **<0.001** | 2.78 (2.08-3.70) | **<0.001** |

**2.7 Prognostic improvement of ASPECTS and NIHSS scales**

A further analysis was performed comparing how N20 response can help to improve the current standard techniques in the assessment of the patient outcome of an endovascular stroke treatment.

The ASPECTS scale (Alberta stroke programme early CT score), is a 10-point quantitative topographic CT scan score used for patients with stroke; where higher values indicate a lesser extent of ischaemia. The NIHSS (National Institutes of Health Stroke Scale), is a systematic assessment tool that provides a quantitative measure of stroke-related neurologic deficit. The higher the values the more severe the stroke is.

Several models were built comparing the good functional prognostic (mRS≤2) performance of these scales as predictors alone (raw) and in combination of the log transformed N20 (adjusted), for both 7-days and 90-days prediction. The AUCs and ROC of the possible 8 models have been compared (see Table 39).

**Table 39:** *Comparison of ASPECTS and NIHSS scales performance with and without log transformed N20 values. In the equations, logBPA represent the log transformed N20 values*

| Model | Response | Variables | Type | AUC (95% CI) | Equation |
|---|---|---|---|---|---|
| A | 7 days | ASPECTS | Raw | 0.64 (0.56-0.71) | $\log\left[\dfrac{P(\text{mrs7}=0\text{-}2)}{1-P(\text{mrs7}=0\text{-}2)}\right]=\alpha+\beta_1(\text{ASPECTS})$ |
| B | 7 days | ASPECTS | Adjusted | 0.76 (0.68-0.84) | $\log\left[\dfrac{P(\text{mrs7}=0\text{-}2)}{1-P(\text{mrs7}=0\text{-}2)}\right]=\alpha+\beta_1(\text{ASPECTS})+\beta_2(\text{logBPA})$ |
| c | 7 days | NIHSS | Raw | 0.67 (0.59-0.75) | $\log\left[\dfrac{P(\text{mrs7}=0\text{-}2)}{1-P(\text{mrs7}=0\text{-}2)}\right]=\alpha+\beta_1(\text{NIHSS\_B})$ |
| D | 7 days | NIHSS | Adjusted | 0.78 (0.70-0.85) | $\log\left[\dfrac{P(\text{mrs7}=0\text{-}2)}{1-P(\text{mrs7}=0\text{-}2)}\right]=\alpha+\beta_1(\text{NIHSS\_B})+\beta_2(\text{logBPA})$ |
| E | 90 days | ASPECTS | Raw | 0.61 (0.53-0.69) | $\log\left[\dfrac{P(\text{mrs90}=0\text{-}2)}{1-P(\text{mrs90}=0\text{-}2)}\right]=\alpha+\beta_1(\text{ASPECTS})$ |
| F | 90 days | ASPECTS | Adjusted | 0.76 (0.67-0.85) | $\log\left[\dfrac{P(\text{mrs90}=0\text{-}2)}{1-P(\text{mrs90}=0\text{-}2)}\right]=\alpha+\beta_1(\text{ASPECTS})+\beta_2(\text{logBPA})$ |
| G | 90 days | NIHSS | Raw | 0.68 (0.60-0.75) | $\log\left[\dfrac{P(\text{mrs90}=0\text{-}2)}{1-P(\text{mrs90}=0\text{-}2)}\right]=\alpha+\beta_1(\text{NIHSS\_B})$ |
| H | 90 days | NIHSS | Adjusted | 0.78 (0.70-0.85) | $\log\left[\dfrac{P(\text{mrs90}=0\text{-}2)}{1-P(\text{mrs90}=0\text{-}2)}\right]=\alpha+\beta_1(\text{NIHSS\_B})+\beta_2(\text{logBPA})$ |

The results show that at 7-days prediction, using NIHSS + N20 (B) instead of NIHSS alone (A) increases AUC from 0.64 to 0.76, and that using ASPECTS + N20 (D) instead of ASPECTS alone (C) increases AUC from 0.67 to 0.78. For the 90-days prediction, the results show that using NIHSS + N20 (F) instead of NIHSS alone (E) increases AUC from 0.61 to 0.76; and that using ASPECTS + N20 (H) instead of ASPECTS alone (G) increases AUC from 0.68 to 0.78.

**3. Conclusions**

Currently, clinical algorithms for the acute ischemic stroke due to large vessel occlusion are mainly based on clinical and neuroimaging predictive factors for the stratification of patient candidates for endovascular treatment. However, the likelihood of significant functional disability or death three months after the stroke still ranges from 40 until 67%. SEPs act as neurophysiological markers and provide additional and different data respect that provided by the rest of predictive factors (NIHSS, blood pressure, hyperglycaemia, ASPECTS, ischemic core and collateral circulation status). It has been observed that SEP intensity values between 0.25 and 0.5µV are the best threshold values to prognosticate the outcome of patients with a high sensitivity, both before, and after the endovascular treatment at 7 and 90-days outcome forecast, according to the mRS scale system. Furthermore, N20 adds predictive power to the clinical variables currently used (NIHSS, ASPECTS), with the effect being slightly more evident in the prognosis at 90 days than at 7 days. Therefore, SEPs could be included in the diagnostic algorithms of the acute ischemic stroke due to large vessel occlusion for improving the indication of endovascular treatment as well as assessing the evolution of the endovascular treatment afterwards.

**Claims**

1. Use of a somatosensory evoked potential, SEP, as a marker for predicting the outcome of an endovascular stroke treatment, wherein the absolute amplitude of a local maximum or minimum of the SEP ipsilateral to the stroke is compared to a first predetermined amplitude threshold value wherein the absolute amplitude of the local maximum or minimum of the SEP ipsilateral to the stroke site above the first predetermined amplitude threshold value is indicative of a good outcome of the endovascular treatment and/or wherein the absolute amplitude of the local maximum or minimum of the SEP ipsilateral to the stroke site below the first predetermined amplitude threshold value is indicative of a bad outcome of the endovascular treatment.

2. The use according to claim 1, wherein the first predetermined amplitude threshold value defines a noise threshold such that the absolute amplitude of the local maximum or minimum of the SEP ipsilateral to the stroke site above the first predetermined amplitude threshold value indicates the presence of the SEP, and/or the absolute amplitude of the local maximum or minimum of the SEP ipsilateral to the stroke site below the first predetermined amplitude threshold value indicates the absence of the SEP, optionally wherein the noise threshold is 0.1 µV.

3. The use according to claim 1, wherein the SEP amplitude of the local maximum or minimum ipsilateral to the stroke site is compared to the first predetermined amplitude threshold value, wherein a SEP amplitude of the local maximum or minimum ipsilateral to the stroke site above the first predetermined amplitude threshold value is indicative of a good outcome of the endovascular treatment.

4. The use according to claim 3, wherein the SEP is the N20 component and the input voltage is the lowest voltage at which the finger visibly twitches and wherein the first predetermined amplitude threshold value is an absolute threshold value and is between 0.1 and 0.75 µV preferably between 0.25 and 0.5 µV, more preferably between 0.3 and 0.4 µV.

5. The use according to any of claims 1, 3 or 4, further comprising comparing the SEP amplitude of the local maximum or minimum ipsilateral to the stroke site to a second predetermined amplitude threshold value, wherein an SEP amplitude of the local maximum or minimum ipsilateral to the stroke site below the second predetermined amplitude threshold value is indicative of a bad outcome of the endovascular treatment.

6. The use according to claim 5, wherein the SEP is the N20 component and the input voltage is the lowest voltage at which the finger visible twitches and wherein the second predetermined amplitude threshold value is an absolute threshold value and is between 0.1 and 0.75 µV, preferably between 0.25 and 0.5 µV, more preferably between 0.3 and 0.4 µV.

7. Use of a somatosensory evoked potential (SEP), as a marker for predicting the outcome of an endovascular treatment, wherein the latency of a local maxima or minima of the SEP ipsilateral to the stroke is compared to a first predetermined latency threshold value, wherein the latency of a local maxima or minima of the SEP ipsilateral to the stroke site below the first predetermined latency threshold value is indicative of a good outcome of the endovascular treatment.

8. The use according to claim 7, further comprising comparing the latency of a local maxima or minima of the SEP ipsilateral to the stroke to a second predetermined latency threshold value, wherein the latency of a local maxima or minima of the SEP ipsilateral to the stroke site above the second predetermined latency threshold value is indicative of a bad outcome of the endovascular treatment.

9. The use according to any preceding claim, wherein the SEP is determined by electrically stimulating the median nerve or the ulnar nerve.

10. The use according to claim 9 wherein the SEP is selected from the N20 and P25 components; preferably wherein the SEP is N20.

11. The use according to any of the claims 1 to 8, wherein the SEP is determined by electrically stimulating the tibial nerve, optionally, wherein the SEP is selected from the N35 and P40 components.

12. The use according to any preceding claim, wherein the amplitude or latency of a local maximum or minimum of the SEP is combined with a quantitative assessment of the stroke severity or one or more clinical variables of the patient.

13. The use according to claim 12, wherein the quantitative assessment is the NIHSS and/or ASPECTS score and/or the collateral circulation state and/or the ischaemia core and/or TICI scale and/or wherein the clinical variables are selected from the age, the sex, the laterality of the stroke, the blood glucose levels and the mean arterial pressure of the patient.

14. An apparatus for predicting the outcome of an endovascular stroke treatment, comprising:

a stimulator for providing electrical stimuli to one or more pairs of stimulating electrodes for stimulating a nerve;
a voltmeter connected to one or more pairs of recording electrodes for recording an SEP resulting from the electrical stimuli provided to the nerve; and
a processor configured to:

compare the absolute amplitude of a local maximum or minimum of the SEP to a predetermined amplitude threshold value wherein the absolute amplitude of the local maximum or minimum of the SEP above the predetermined amplitude threshold value is indicative of a good outcome of the endovascular treatment and/or wherein the absolute amplitude of the local maximum or minimum of the SEP below the first predetermined amplitude threshold value is indicative of a bad outcome of the endovascular treatment; and/or
compare the latency of a local maxima or minima of the SEP to a predetermined latency threshold value, wherein the latency of a local maxima or minima of the SEP ipsilateral to the stroke site below the first predetermined latency threshold value is indicative of a good outcome of the endovascular treatment.

Fig. 1

Fig. 2

**Fig. 3A**. NIHSS. AUC=0.670

**Fig. 3B**. N20. AUC=0.726

Fig.3 (cont.)

**Fig. 3C**. NIHSS+ N20=0.784

**Fig. 3D**. NIHSS+ASPECTS. AUC=0.719

**Fig.3 (cont.)**

**Fig. 3E.**
NIHSS+Baseline variables=0.750

**Fig. 3F.**
NIHSS+Baseline variables+N20=0.823

Fig. 4A.
N20=0.737

Fig. 4B.
NIHSS+ASPECTS.AUC=0.697

Fig.4 (cont.)

Fig. 4D.
NIHSS+Baseline variables+N20.AUC=0.851

Fig. 4C.
NIHSS+Baseline variables.AUC=0.793

**Fig. 5A.** TICI. AUC=0.620

**Fig. 5B.** N20 (log-transformed). AUC=0.823

**Fig. 5C.** TICI+N20(log-transformed).AUC=0.829

**Fig. 6A.** TICI. AUC=0.626

**Fig. 6B.** N20 (log-transformed). AUC=0.917

**Fig. 6C.** TICI+N20 (log-transformed). AUC=0.919

100

105

| | | |
|---|---|---|
| 110 | 140 | |
| 120 | 150 | 155 |
| 130 | 160 | |

135

145

200

FIG 7

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/113462 A1 (MARTÍNEZ PIÑEIRO ALICIA [ES] ET AL) 16 April 2020 (2020-04-16) * paragraph [0027] – paragraph [0068] * * paragraph [0093] – paragraph [0103] * * figures * | 1-6,9-14 | INV. A61B5/383 A61B5/00 |
| X | B D ZEMAN ET AL: "Functional prognosis in stroke: use of somatosensory evoked potentials.", JOURNAL OF NEUROLOGY NEUROSURGERY & PSYCHIATRY., vol. 52, no. 2, 1 February 1989 (1989-02-01), pages 242-247, XP055395841, GB ISSN: 0022-3050, DOI: 10.1136/jnnp.52.2.242 | 1-10,14 | |
| A | * page 243, left-hand column, paragraph 4 – right-hand column, paragraph 4 * * page 243, right-hand column, last paragraph – page 244, left-hand column, paragraph 1 * * page 246, left-hand column, last paragraph – right-hand column, paragraph 1 * | 11-13 | |

-/--

TECHNICAL FIELDS
SEARCHED      (IPC)

A61B

The present search report has been drawn up for all claims

| Place of search | Date of completion of the search | Examiner |
|---|---|---|
| The Hague | 8 July 2022 | Görlach, Tobias |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................
& : member of the same patent family, corresponding
document

EPO FORM 1503 03.82 (P04C01)

page 1 of 2

## EUROPEAN SEARCH REPORT

Application Number

EP 22 38 2090

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TZVETANOV P ET AL: "Prognostic value of median and tibial somatosensory evoked potentials in acute stroke", NEUROSCIENCE LETTERS, ELSEVIER, AMSTERDAM, NL, vol. 380, no. 1-2, 20 May 2005 (2005-05-20), pages 99-104, XP027653613, ISSN: 0304-3940 [retrieved on 2005-05-20] * page 99, left-hand column, paragraph 1 * * page 100, right-hand column, last paragraph – page 101, right-hand column, last paragraph * | 1-14 | |
| | | | **TECHNICAL FIELDS SEARCHED** (IPC) |

The present search report has been drawn up for all claims

| Place of search | Date of completion of the search | Examiner |
|---|---|---|
| The Hague | 8 July 2022 | Görlach, Tobias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 38 2090

08-07-2022

| Patent document cited in search report | | Publication date | Patent family member(s) | | Publication date |
|---|---|---|---|---|---|
| US 2020113462 | A1 | 16-04-2020 | CA | 3061078 A1 | 23-08-2018 |
| | | | EP | 3363356 A1 | 22-08-2018 |
| | | | EP | 3582685 A1 | 25-12-2019 |
| | | | US | 2020113462 A1 | 16-04-2020 |
| | | | WO | 2018149973 A1 | 23-08-2018 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• **DAVALOS A et al.** *Lancet Neurol,* 2017